# EUROPEAN PATENT SPECIFICATION

(11) **EP 4 394 525 B1**
(45) Date of publication and mention of the grant of the patent: **27.08.2025**
(21) Application number: 22887284.2
(22) Date of filing: 07.07.2022
(51) Int. Cl.: G04G 21/02, G01N 21/25, A61B 5/024, A61B 5/1455, A61B 5/021, A61B 5/00

(54) **ELECTRONIC DEVICE COMPRISING STRUCTURE FOR FACILITATING OPERATION OF PLURALITY OF SENSORS HAVING DIFFERENT OPTICAL CHARACTERISTICS**
ELEKTRONISCHE VORRICHTUNG MIT STRUKTUR ZUR ERLEICHTERUNG DES BETRIEBS MEHRERER SENSOREN MIT VERSCHIEDENEN OPTISCHEN EIGENSCHAFTEN
DISPOSITIF ÉLECTRONIQUE COMPRENANT UNE STRUCTURE DESTINÉE À FACILITER LE FONCTIONNEMENT D'UNE PLURALITÉ DE CAPTEURS PRÉSENTANT DES CARACTÉRISTIQUES OPTIQUES DIFFÉRENTES

(30) Priority: 25.10.2021 KR 20210142477; 22.11.2021 KR 20210161267
(43) Date of publication of application: 03.07.2024
(73) Proprietor: Samsung Electronics Co., Ltd., Suwon-si, Gyeonggi-do 16677 (KR)
(72) Inventor: PARK, Minho, Suwon-si Gyeonggi-do 16677 (KR); JEONG, Injo, Suwon-si Gyeonggi-do 16677 (KR); KIM, Younghyun, Suwon-si Gyeonggi-do 16677 (KR)
(74) Representative: HGF
(86) International application number: PCT/KR2022/009854
(87) International publication number: WO 2023/075078

(56) References cited:
- JP-A- 2020 513 876
- KR-A- 20160 028 303
- KR-A- 20190 119 382
- KR-A- 20200 093 247
- KR-A- 20210 087 793
- US-A1- 2013 046 192
- US-A1- 2016 073 954

## Description

### [Technical Field]

One or more embodiments relate to an electronic device including a structure for facilitating operations of a plurality of sensors having different optical characteristics.

### [Background Art]

An electronic device may be configured to obtain biometric information of a user and notify the user of the obtained biometric information. The electronic device for obtaining biometric information may be a wearable device that is worn by the user and contacts with a part of the user's body. For example, the wearable device may be worn on the user's finger, ankle, wrist, ear, or face.

In order to conveniently obtain the user's biometric information, the electronic device may include an optical sensor. For example, the electronic device may include a photoplethysmogram (PPG) sensor that emits light to a part of the human body and receives light reflected from the body. As capabilities of the electronic device have proliferated, the electronic device may include various types of optical sensors in addition to the PPG sensor. Relevant prior art is disclosed in US 2016/0073954 A1, US 2013/0046192 A1, JP 2020 513876 A, KR 2020 0093247 A, KR 2019 0119382 A, KR 2021 0087793 A, KR 2016 0028303 A.

### [Disclosure]

### [Technical Problem]

When the user wears an electronic device, the electronic device may contact, for example, the user's wrist. As capabilities of the electronic device have proliferated, the electronic device may include a plurality of different types of optical sensors. Since the plurality of optical sensors have different optical characteristics, performance of these various sensors may deteriorate due to crosstalk of light when the sensors are disposed adjacent to each other. Thus, the plurality of optical sensors may need to be disposed in different areas of the electronic device to optimize performance. But because the electronic device is miniaturized so that it can be worn on the user's wrist, the space for disposing various sensors in the electronic device may be limited. The electronic device may thus require novel ways of disposing a plurality of sensors having different optical characteristics within limited space.

One or more embodiments disclosed herein generally relate to an electronic device including a structure for facilitating operations of a plurality of sensors having different optical characteristics. The technical problems to be achieved in this document are not limited to those described above, and other technical problems not mentioned herein will be clearly understood by those having ordinary knowledge in the art to which the present disclosure belongs, from the following description.

### [Technical Solution]

According to the invention, as defined in claim 1, an electronic device may comprise a housing, a printed circuit board disposed within the housing, a window including a first region parallel to the printed circuit board and a second region connecting a periphery of the first region and the housing, the window facing a part of a user's body when the electronic device is worn by the user, a PPG (photoplethysmogram) sensor disposed on the printed circuit board and including at least one first emitting portion configured to emit light at a first beam angle toward the window and at least one first receiving portion configured to receive light emitted from the at least one first emitting portion and reflected from the part of the user's body, a laser sensor including at least one second emitting portion configured to emit light at a second beam angle smaller than the first beam angle toward the window, and at least one second receiving portion configured to receive light emitted from the at least one second emitting portion and reflected from the part of the user's body and an index layer disposed on the laser sensor; wherein the laser sensor may be disposed on the printed circuit board to be surrounded by the PPG sensor and may overlap the first region, when the window is viewed from an outside of the electronic device. In addition, various embodiments may be possible.

According to an embodiment, an electronic device may comprise a housing including a first surface, a second surface opposite the first surface and an inner space formed between the first surface and the second surface, a printed circuit board disposed in the inner space, a window forming at least part of the second surface of the housing, and including a first region parallel to the printed circuit board and configured to contact a part of a user's body when the electronic device is worn by the user, and a second region having curvature and connecting a periphery of the first region and the housing, a PPG (photoplethysmogram) sensor disposed on the printed circuit board and including at least one first emitting portion configured to emit light at a first beam angle toward the window and at least one first receiving portion configured to receive light emitted from the at least one first emitting portion and reflected from the part of the user's body, a laser sensor including at least one second emitting portion configured to emit light at a second beam angle smaller than the first beam angle toward the window, and at least one second receiving portion configured to receive light emitted from the at least one second emitting portion and reflected from the part of the user's body, an index layer disposed on the laser sensor and a barrier disposed on the laser sensor to be positioned between one region of the window overlapping the at least one second emitting portion and another region of the window overlapping the at least one second receiving portion when the window is viewed from the outside of the electronic device, wherein the laser sensor may be disposed on the printed circuit board to be surrounded by the PPG sensor and may overlap the first region when the window is viewed from an outside of the electronic device.

### [Advantageous Effects]

According to an embodiment, as a laser sensor having optical characteristics different from that of a PPG sensor is disposed within a housing to be surrounded by the PPG sensor, an electronic device can arrange the PPG sensor and the laser sensor within a limited arrangement space, and secure performance of the PPG sensor and the laser sensor. As the performance of the PPG sensor and the laser sensor is secured, the electronic device can obtain various biometric information from a user and notify the user of the obtained biometric information.

The effects that can be obtained from the present disclosure are not limited to those described above, and any other effects not mentioned herein will be clearly understood by those having ordinary knowledge in the art to which the present disclosure belongs, from the following description.

### [DESCRIPTION OF THE DRAWINGS]

FIG. 1 is a block diagram of an electronic device in a network environment according to an embodiment.
FIGS. 2A and 2B are perspective views of an electronic device according to an embodiment.
FIG. 3 is an exploded perspective view of an electronic device according to an embodiment.
FIG. 4 is a perspective view of a second surface of an electronic device according to an embodiment.
FIG. 5A is a top view illustrating an example of an arrangement relationship between a PPG sensor and a laser sensor of an electronic device according to an embodiment.
FIG. 5B is a top view illustrating other example of an arrangement relationship between a PPG sensor and a laser sensor of an electronic device according to an embodiment.
FIG. 5C is a top view illustrating yet another example of an arrangement relationship between a PPG sensor and a laser sensor of an electronic device according to an embodiment.
FIG. 6 is a diagram illustrating an example in which an electronic device is cut along A-A' of FIG. 4, according to an embodiment.
FIG. 7A is a cross-sectional view illustrating a cross-section of an electronic device according to an embodiment.
FIG. 7B is a plan view of a second surface of an electronic device, according to an embodiment.
FIG. 8 is a cross-sectional view illustrating a cross-section of an electronic device according to an embodiment.
FIG. 9 is a cross-sectional view illustrating a cross-section of an electronic device according to an embodiment.
FIG. 10 is a cross-sectional view illustrating a cross-section of an electronic device according to an embodiment.
FIG. 11 is a cross-sectional view illustrating a cross-section of an electronic device according to an embodiment.

### [Mode for Invention]

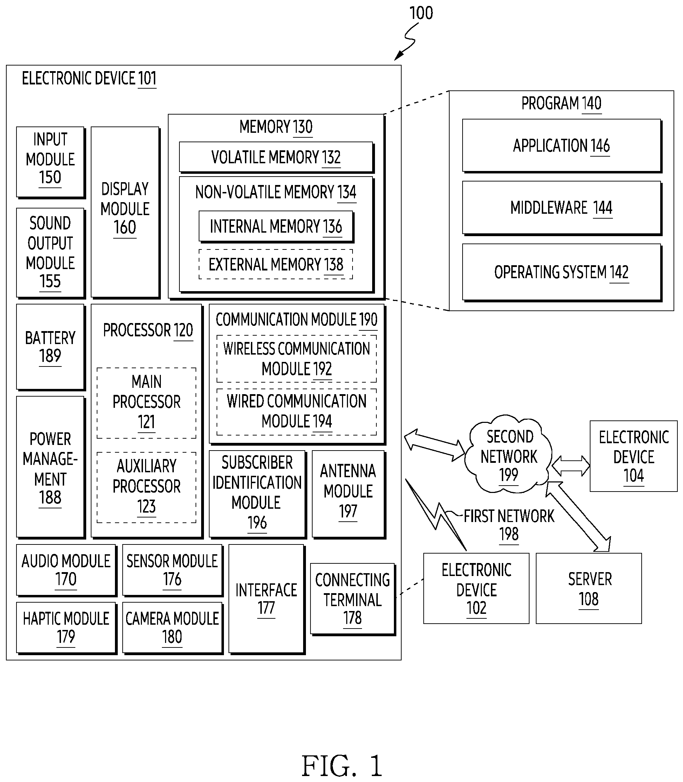

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

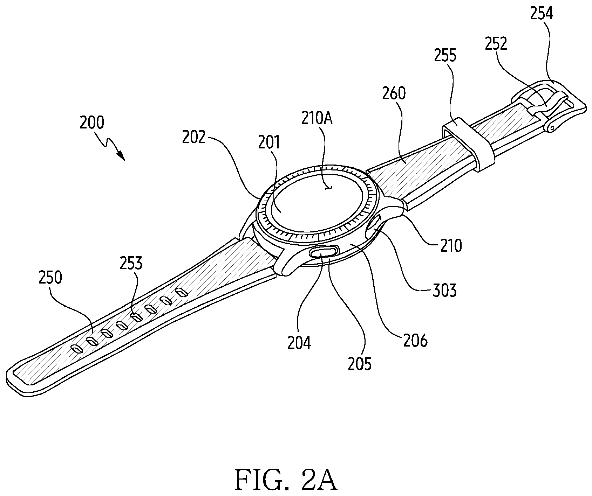

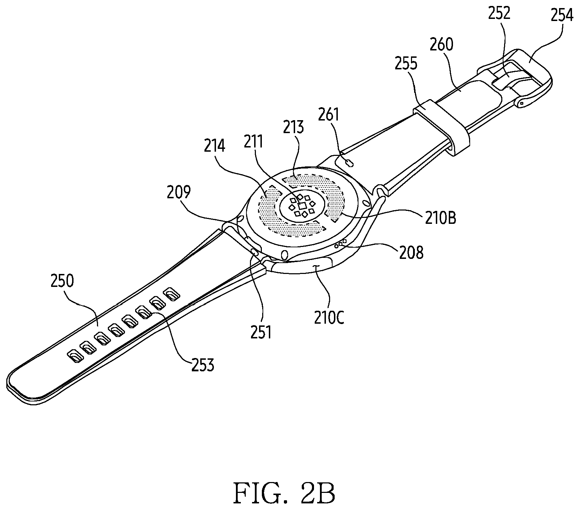

FIGS. 2A and 2B are perspective views of an electronic device according to an embodiment.

Referring to FIGS. 2A and 2B, an electronic device 200 (e.g., the electronic device 101 of FIG. 1) according to an embodiment may include a housing 210 including a first surface (or front surface) 210A, a second surface (or rear surface) 210B, and a side surface 210C surrounding the space between the first surface 210A and the second surface 210B and binding members 250 and 260 connected to at least a part of the housing 210 and configured to detachably attach the electronic device 200 to a part of the user's body (e.g., wrist, ankle, etc.). In another embodiment (not illustrated), the housing may also refer to a structure that forms at least a part of the first surface 210A, the second surface 210B, and the side surface 210C of FIG. 2A. According to an embodiment, at least a part of the first surface 210A may be implemented by a substantially transparent front plate 201 (e.g., glass plate or polymer plate including various coating layers). The second surface 210B may be implemented by a substantially opaque rear plate 207. The rear plate 207 may be made of, for example, coated or colored glass, ceramic, polymer, metal (e.g., aluminum, stainless steel (STS), or magnesium), or a combination of at least two of the materials. The side surface 210C may be coupled to the front plate 201 and the rear plate 207, and may be implemented by a side bezel structure (or "side member") 206 including metal and/or polymer. In some embodiments, the rear plate 207 and the side bezel structure 206 may be integrally formed and may include the same material (e.g., metal material such as aluminum). The binding members 250 and 260 may be made of various materials and may be made in various shapes. The binding members 250 and 260 may be made of woven fabric, leather, rubber, urethane, metal, ceramic, or a combination of at least two of the materials.

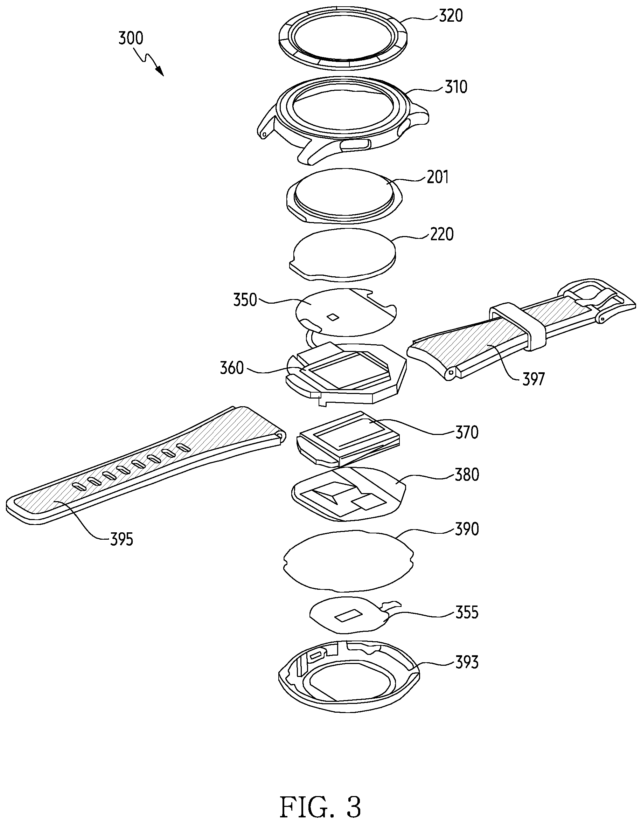

According to an embodiment, the electronic device 200 may include at least one of a display 220 (see FIG. 3), an audio module 205 and 208, a sensor module 211, a key input device 202, 203 and 204, and a connector hole 209. In some embodiments, the electronic device 200 may omit at least one of the components (e.g., the key input devices 202, 203 and 204, the connector hole 209, or the sensor module 211) or may further include another component.

The display 220 may be exposed, for example, through a substantial portion of the front plate 201. The shape of the display 220 may correspond to the shape of the front plate 201, such as circular (shown in FIG. 2), oval, or polygonal. The display 220 may be coupled to or adjacent to a touch sensing circuit, a pressure sensor capable of measuring the strength (pressure) of touches, and/or a fingerprint sensor.

The audio modules 205 and 208 may include a microphone hole 205 and a speaker hole 208. A microphone for obtaining external sound may be disposed inside the microphone hole 205, and in some embodiments, a plurality of microphones may be disposed to detect the direction of the sound. The speaker hole 208 may be used with an external speaker and a receiver for phone calls. In some embodiments, the speaker hole 208 and the microphone hole 205 may be implemented as a single hole, or a speaker (e.g., piezo speaker) may be included without the speaker hole 208.

The sensor module 211 may generate electrical signal(s) or data value(s) corresponding to internal operating state(s) of the electronic device 200 or external environmental state(s). The sensor module 211 may include, for example, a biometric sensor module 211 (e.g., heart-rate monitor (HRM) sensor) disposed on the second surface 210B of the housing 210. The electronic device 200 may further include at least one sensor module not shown, such as a gesture sensor, a gyro sensor, a pressure sensor, a magnetic sensor, an acceleration sensor, a grip sensor, a color sensor, an infrared sensor, a biometric sensor, a humidity sensor, and/or an illumination sensor.

The sensor module 211 may include electrode regions 213 and 214 forming a part of the surface of the electronic device 200 and a bio-signal detection circuit (not shown) electrically connected to the electrode regions 213 and 214. For example, the electrode regions 213 and 214 may include the first electrode region 213 and the second electrode region 214 disposed on the second surface 210B of the housing 210. The sensor module 211 may be configured such that the electrode regions 213 and 214 obtain electrical signal(s) from a part of the user's body, and the bio-signal detection circuit may detect biometric information of the user based on the electrical signal(s).

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key may have a shape corresponding to the shape of the front plate 201. In another embodiment, the electronic device 200 may not include some or all of the above-described key input devices 202, 203, and 204, and the not included key input devices 202, 203, and 204 may be implemented in other forms such as soft keys on the display 220. The connector hole 209 may accommodate a connector (e.g., USB connector) for transmitting and receiving power and/or data to and from external electronic devices and may include another connector hole (not illustrated) capable of accommodating a connector for transmitting and receiving audio signals to and from an external electronic device. The electronic device 200 may further include, for example, a connector cover (not illustrated) that covers at least a part of the connector hole 209 and blocks the inflow of external foreign material into the connector hole.

The binding members 250 and 260 may be detachably attached to at least a part of the housing 210 using locking members 251, 261. The binding members 250 and 260 may include one or more of a fixing member 252, a fixing member fastening hole 253, a band guide member 254, and a band fixing ring 255.

The fixing member 252 may be configured to fix the housing 210 and the binding members 250 and 260 to a part of the user's body (e.g., wrist, ankle, etc.). The fixing member fastening hole 253 may correspond to the fixing member 252 to fix the housing 210 and the binding members 250 and 260 to the part of the user's body. The band guide member 254 may be configured to limit movement range of the fixing member 252 when the fixing member 252 is fastened to the fixing member fastening hole 253, so that the binding members 250 and 260 are attached to be in close contact with the part of the user's body. The band fixing ring 255 may limit the range of movement of the fixing members 250 and 260 when the fixing member 252 and the fixing member fastening hole 253 are fastened. FIG. 3 is an exploded perspective view of an electronic device according to an embodiment.

Referring to FIG. 3, an electronic device 300 (e.g., the electronic device 101 of FIG. 1, the electronic device 200 of FIG. 2A and/or FIG. 2B) may include a side bezel structure 310, a wheel key 320, a front plate 201, a display 220, a first antenna 350, a second antenna 355, a support member 360 (e.g., bracket), a battery 370, a printed circuit board 380, a sealing member 390, and binding members 395 and 397. At least one of the components of the electronic device 300 may be the same as or similar to at least one of the components of the electronic device 200 of FIGS. 1, 2A, and/or 2B, and repeated description thereof will be omitted. The support member 360 may be disposed inside the electronic device 300 to be connected to the side bezel structure 310 or may be integrated with the side bezel structure 310. The support member 360 may be made of, for example, metal material and/or non-metal (e.g., polymer) material. The display 220 may be coupled to one surface of the support member 360, and the printed circuit board 380 may be coupled to the other surface of the support member 360. A processor, a memory, and/or an interface may be mounted on the printed circuit board 380. The processor may include, for example, one or more of a central processing unit, an application processor, a graphic processing unit (GPU), an application processor, a sensor processor, or a communication processor.

The memory may include, for example, a volatile memory or a nonvolatile memory. The interface may include, for example, a high definition multimedia interface (HDMI), a universal serial bus (USB) interface, an SD card interface, and/or an audio interface. The interface may electrically or physically connect the electronic device 300 to an external electronic device, for example, and may include a USB connector, an SD card/MMC connector, or an audio connector.

The battery 370 is a device for supplying power to at least one component of the electronic device 300, and may include, for example, a non-rechargeable primary battery, a rechargeable secondary battery, or a fuel battery. At least a part of the battery 370 may be disposed on substantially the same plane as, for example, the printed circuit board 380. The battery 370 may be integrally disposed inside the electronic device 200 or may be detachably coupled to the electronic device 200.

The first antenna 350 may be disposed between the display 220 and the support member 360. The first antenna 350 may include, for example, a near field communication (NFC) antenna, a wireless charging antenna, and/or a magnetic secure transmission (MST) antenna. The first antenna 350 may, for example, perform short-range communication with an external device, wirelessly transmit and receive power required for charging, and transmit short-range communication signal or an electromagnetic signal including payment data. In another embodiment, an antenna structure may be formed by at least a portion of the side bezel structure 310 and/or a part of the support member 360 or a combination thereof. The second antenna 355 may be disposed between the printed circuit board 380 and the rear plate 393. The second antenna 355 may include, for example, a near field communication (NFC) antenna, a wireless charging antenna, and/or a magnetic secure transmission (MST) antenna. For example, the second antenna 355 may perform short-range communication with an external device, wirelessly transmit and receive power required for charging, and may transmit a short-range communication signal or a electromagnetic signal including payment data. In another embodiment, an antenna structure may be formed by at least a portion of the side bezel structure 310 and/or a part of the rear plate 393 or a combination thereof.

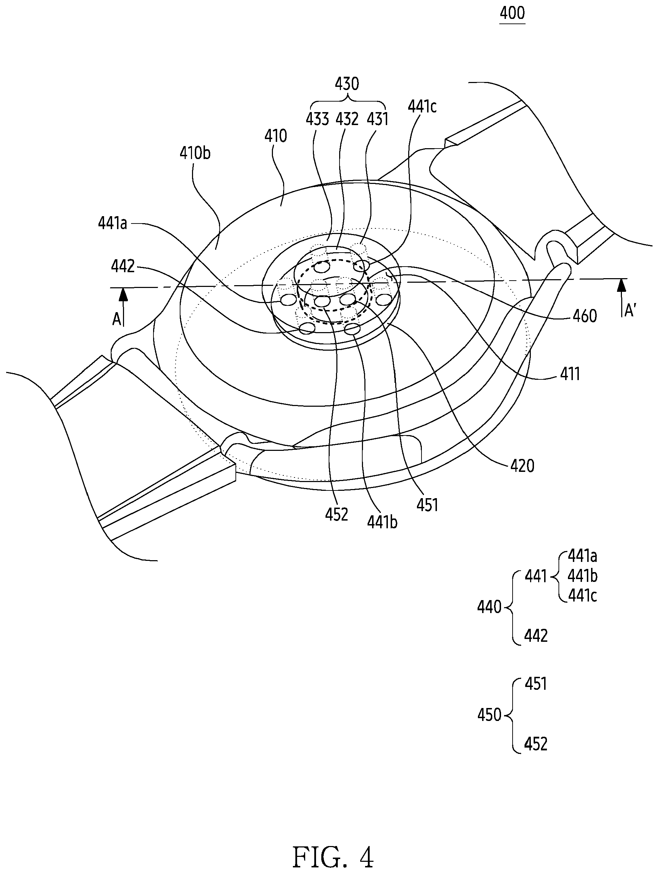

The sealing member 390 may be positioned between the side bezel structure 310 and the rear plate 393. The sealing member 390 may be configured to block moisture and foreign material flowing into the space surrounded by the side bezel structure 310 and the rear plate 393 from the outside. FIG. 4 is a perspective view of a second surface of an electronic device according to an embodiment.

Referring to FIG. 4, the electronic device 400 according to an embodiment may include a housing 410, a printed circuit board 420, a window 430, a PPG sensor 440, a laser sensor 450, and an index layer 460. According to an embodiment, the electronic device 400 may be referred to as the electronic device 101 of FIG. 1, the electronic device 200 of FIG. 2A and/or 2B, and/or the electronic device 300 of FIG. 3. According to an embodiment, when worn by the user, the electronic device 400 may contact a part of the user's body. For example, when the electronic device 400 is worn by the user, a part of the second surface 410b of the electronic device 400 may contact at least a part of the user's wrist.

The housing 410 may provide the overall outer shape of the electronic device 400. According to an embodiment, the housing 410 may include a first surface (e.g., the first surface 210A in FIG. 2A) and a second surface 410b facing the first surface 210A. The second surface 410b may be substantially the same as the second surface 210B of FIG. 2B. According to an embodiment, when the electronic device 400 is worn by the user, the second surface 410b may face or be in contact with a part of the user's body. According to an embodiment, the housing 410 may include an inner space 411 where various components of the electronic device 400 are disposed. For example, the inner space 411 may be bounded by a side surface (e.g., the side surface 210c of FIG. 2A) extending from the peripheries of the first surface 210A and the second surface 410b, the first surface 210A, and the second surface 410b.

The printed circuit board 420 may be electrically connected to various components of the electronic device 400 that perform various operations of the electronic device 400. According to an embodiment, the printed circuit board 420 may be disposed in the inner space 411 formed by the housing 410. According to an embodiment, the printed circuit board 420 may be electrically connected to various electronic components disposed on the printed circuit board 420 or various components of the electronic device 400 disposed outside the printed circuit board 420. According to an embodiment, the printed circuit board 420 may be electrically connected to various components of the electronic device 400 through a connection member (not illustrated). For example, the connection member may include a coaxial cable connector, a board to board connector, an interposer, or a flexible printed circuit board (FPCB).

The window 430 may protect internal components of the electronic device 400. According to an embodiment, the window 430 may be coupled to the housing 410 to protect the printed circuit board 420 in the housing 410. For example, the window 430 may form a part of the second surface 410b of the housing 410. According to an embodiment, at least a part of the window 430 may be configured to be transparent so as to transmit light. For example, at least a part of the window 430 may be made of a substantially transparent material to transmit light, and the housing 410 surrounding the window 430 may be made of a substantially opaque material to prevent light transmission.

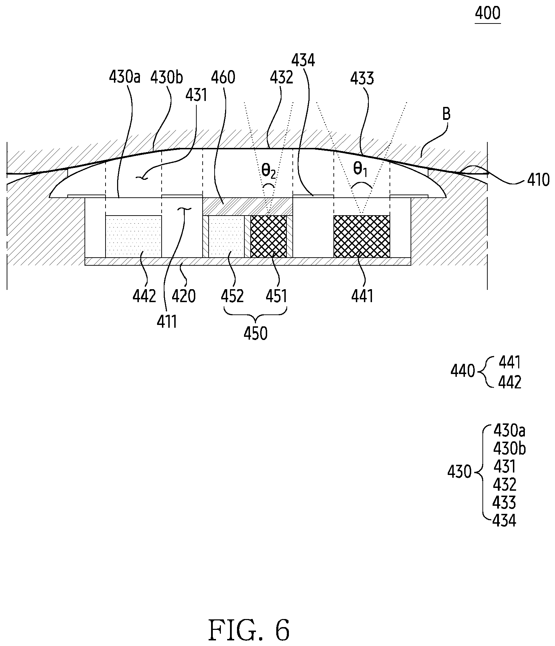

According to an embodiment, the window 430 may include a plurality of openings 431. The plurality of openings 431 may be regions of the window 430 configured to transmit light. For example, the window 430 may include material that absorbs light in region(s) other than the regions corresponding to the plurality of openings 431. As the material absorbing light is disposed within the window 430, the region(s) other than the regions corresponding to the plurality of openings 431 may not transmit light. According to an embodiment, a layer (e.g., the absorption layer 434 of FIG. 6) including light blocking material (e.g. light absorbing material) may be applied under the window 430. For example, the plurality of openings 431 may form paths through which light travels into the housing 410, because light blocking material is disposed in region(s) of the layer not corresponding to the plurality of openings 431 and the light blocking material is not disposed in regions of the layer corresponding to the plurality of openings 431.

According to an embodiment, the window 430 may include a first region 432 and a second region 433. The first region 432 may be substantially parallel to the printed circuit board 420. For example, the printed circuit board 420 may have a planar shape extending in the inner space 411, and the first region 432 may have a planar shape substantially parallel to the printed circuit board 420. The second region 433 may connect the first region 432 and the housing 410. According to an embodiment, the second region 433 may surround the first region 432. A part of the housing 410 forming the second surface 410b may be outside the second region 433 and surround the second region 433. For example, the second region 433 may be formed to have curvature unlike the first region 432, but is not limited thereto, and the second region 433 may be substantially parallel to the printed circuit board 420 similar to the first region 432.

According to an embodiment, the window 430 may be spaced apart from the printed circuit board 420. As the window 430 is spaced apart from the printed circuit board 420, components (e.g., PPG sensor 440 and laser sensor 450) disposed on the printed circuit board 420 may be protected. When the window 430 and the printed circuit board 420 directly contact each other, components disposed on the printed circuit board 420 and the printed circuit board 420 may be damaged by impacts applied from the outside of the electronic device 400. As the window 430 is spaced apart from the printed circuit board 420, the impacts applied from the outside of the electronic device 400 may be attenuated when they reach the printed circuit board 420. The space formed between the window 430 and the printed circuit board 420 may accommodate various components of the electronic device 400.

The PPG sensor 440 may obtain first biometric data related to the user's body. The first biometric data may include at least one of heart rate, a change in the user's heart rate during a preset time interval, oxygen saturation, and blood pressure. According to an embodiment, when the electronic device 400 is worn by the user, the PPG sensor 440 may face or be in contact with a part of the user's body. The PPG sensor 440 may be configured to emit light to the user's body and receive light reflected from the user's body in order to detect a change in the amount of blood flow inside the microvasculature of the user's body. In the user's body, the volume of the microvasculature may be changed by changing the amount of blood flow in the microvasculature due to the periodic contraction or relaxation of the heart. The degree to which light transmitted to the user's body is absorbed into the body may vary according to the change in the amount of blood flow of the microvasculature. The PPG sensor 440 may obtain first biometric data based on the intensity of light reflected from the user's body.

According to an embodiment, the PPG sensor 440 may be disposed on the printed circuit board 420 in the housing 410. According to an embodiment, the PPG sensor 440 may include at least one first light emitting portion 441 and at least one first receiving portion 442. The first light emitting portion 441 and the first receiving portion 442 may correspond to each other. For example, the number of the first light emitting portion 441 and the number of the first receiving portion 442 may be the same, and the first light emitting portion 441 and the first receiving portion 442 having the same number may constitute a PPG sensor 440. For another example, the number of the first light emitting portion 441 and the number of the first receiving portion 442 may be different from each other, and the first light emitting portion 441 and the first receiving portion 442 having different numbers may constitute a PPG sensor 440.

The first light emitting portion 441 may be configured to emit light to the part of the user's body in contact with the electronic device 400 when the electronic device 400 is worn by the user. The first light emitting portion 441 may be configured to emit light to the part of the user's body in a first beam angle. For example, the first beam angle may be about 120 degrees, but is not limited thereto. The first light emitting portion 441 may be, for example, a light emitting diode (LED), but is not limited thereto. For example, the first light emitting portion 441 may include a laser light source (e.g., laser diode (LD) and/or solid laser) emitting laser. According to an embodiment, there are the plurality of first light emitting portions 441, and the plurality of first light emitting portions 441a, 441b, and 441c may emit light having different wavelengths. According to an embodiment, the first light emitting portions 441a, 441b, and 441c may emit light in the visible band. For example, the first light emitting portions 441a, 441b, and 441c may emit green light to measure the user's heart rate, stress, or blood pressure, or red light to measure the user's oxygen saturation. According to an embodiment, the total wavelength bandwidth of light emitted by the plurality of first light emitting portions 441a, 441b, and 441c may be about 500 nm to about 1000 nm, but it is not limited thereto.

The first receiving portion 442 may be configured to receive light emitted from the first light emitting portion 441 and reflected from a part of the user's body. The first receiving portion 442 may generate an electrical signal corresponding to the light reflected from the part of the body. The electrical signal generated by the first receiving portion 442 may be transmitted to the processor (e.g., the processor 120 of FIG. 1) through the printed circuit board 420. For example, the first receiving portion 442 may refer to a photo diode, but is not limited thereto. According to an embodiment, the first receiving portion 442 may be spaced apart from the first light emitting portion 441 and disposed on the printed circuit board 420. As the receiving portion 442 and the first light emitting portion 441 are spaced apart from each other, crosstalk between light emitted from the first light emitting portion 441 and light received by the first receiving portion 442 may be reduced.

The laser sensor 450 may obtain second biometric data related to the user's body. For example, the second biometric data may be the blood sugar level in the user's body or blood alcohol content. According to an embodiment, when the electronic device 400 is worn by the user, the laser sensor 450 may face or be in contact with a part of the user's body. The laser sensor 450 may be configured to emit light to the user's body and receive light reflected from the user's body. For example, light emitted from the laser sensor 450 may penetrate the user's body to reach some particular material in the user's body (e.g., glucose in a blood vessel or alcohol molecule in a blood vessel), change wavelength or intensity, or generate vibration. The laser sensor 450 may obtain the second biometric data based on the wavelength, intensity, or vibration of light reflected from the user's body.

According to an embodiment, the laser sensor 450 may include at least one second light emitting portion 451 and at least one second receiving portion 452. The at least one second light emitting portion 451 and the second receiving portion 452 may correspond to each other. For example, the number of the second light emitting portion 451 and the number of the second receiving portion 452 may be the same, and the second light emitting portion 451 and the second receiving portion 452 having the same number may constitute a laser sensor 450. For another example, the number of second light emitting portions 451 and the number of second receiving portion 452 are different from each other, and the second light emitting portion 451 and the second receiving portion 452 having different numbers may constitute a laser sensor 450.

According to an embodiment, the second light emitting portion 451 may be configured to emit light to the part of the user's body in contact with the electronic device 400 when worn by the user. According to an embodiment, the light emitted by the second light emitting portion 451 of the laser sensor 450 may have higher directivity than the light emitted by the first light emitting portion 441 of the PPG sensor 440. The second light emitting portion 451 may be configured to emit light having second beam angle smaller than the first beam angle, to the part of the user's body. For example, the second beam angle may be about 5 degrees, but is not limited thereto. According to an embodiment, the second light emitting portion 451 may emit light in the infrared band that includes a near infrared region. For example, there may be plurality of second light emitting portions 451, and each of the plurality of second light emitting portions 451 may be a laser light source emitting light of different wavelengths. The plurality of second light emitting portions 451 may form a laser array. For example, the total wavelength bandwidth of light emitted by the plurality of second light emitting portions 451 may be approximately 1200 nm to 2400 nm, but is not limited thereto.

According to an embodiment, since the second light emitting portion 451 includes a laser modulator, the number of second light emitting portion 451 may not correspond to the number light beams having different characteristics emitted from the second light emitting portion 451. The second light emitting portion 451 may emit laser beams of different characteristics without requiring laser light sources each dedicated to a particular beam by, for example, changing the wavelength of the laser emitted from a single laser light source by using the laser modulator. For example, when the second light emitting portion 451 includes one laser light source, the second light emitting portion 451 may sequentially change the wavelength of the laser emitted from the one laser light source by using the modulator to emit laser beams of different characteristics from the one laser light source. In another example, when the second light emitting portion 451 includes a plurality of laser light sources, the second light emitting portion 451 may simultaneously emit laser beams of different characteristics by changing the wavelengths of the lasers emitted from the plurality of laser light sources by using the modulator. The second receiving portion 452 may be configured to receive light emitted from the second light emitting portion 451 and reflected from the part of the user's body. The second receiving portion 452 may generate an electrical signal corresponding to the light reflected from the part of the body. The electrical signal generated by the second receiving portion 452 may be transmitted to the processor (e.g., the processor 120 of FIG. 1) through the printed circuit board 420. For example, the second receiving portion 452 may be implemented as a photodiode but is not limited thereto. According to an embodiment, the second receiving portion 452 may be spaced apart from the second light emitting portion 451 and disposed on the printed circuit board 420. As the second receiving portion 452 and the second light emitting portion 451 are spaced apart from each other, crosstalk between light emitted by the second light emitting portion and light received by the second receiving portion may be decreased. According to an embodiment, since the light emitted by the second light emitting portion 451 of the laser sensor 450 has stronger directivity than the light emitted by the first light emitting portion 441 of the PPG sensor 440, the distance between the second light emitting portion 451 and the second receiving portion 452 may be smaller than the distance between the first light emitting portion 441 and the first receiving portion 442.

According to an embodiment, at least one second light emitting portion 451 may include a plurality of second light emitting portions 451 that emit light having different wavelengths and at least one first light emitting portion 441 may include a plurality of first light emitting portions 441a, 441b, and 441c that emit light having different wavelengths. The wavelength range of light emitted by the plurality of second light emitting portions 451 may be narrower than the wavelength range of light emitted by the plurality of first light emitting portions 441. According to an embodiment, the plurality of second light emitting portions 451 may be a plurality of laser light sources emitting light having different wavelengths, and each of the plurality of first light emitting portions 441a, 441b, and 441c may be an LED. When an LED configured to emit light having a designated wavelength, since light emitted from the LED has monochromaticity lower than that of a laser, the wavelength range of light emitted from the LED may be wider than the wavelength range of light emitted from the laser light source. For example, the wavelength range of light emitted from LEDs configured to emit light of wavelength of 500 nm may be approximately 450 nm to 550 nm, but the wavelength range of light emitted from a laser light source configured to emit light having wavelength of 1300 nm may be approximately 1299 nm to 1301 nm.

According to an embodiment, the laser sensor 450 may be disposed on the printed circuit board 420 to be surrounded (or encircled) by the PPG sensor 440. For example, at least one first light emitting portion 441 and at least one first receiving portion 442 forming the PPG sensor 440 may surround the periphery of the laser sensor 450. For example, at least one first light emitting portion 441 may include a plurality of first light emitting portions 441a, 441b, and 441c that emit light with different wavelengths for the electronic device 400 to obtain various first biometric information. Since each of the plurality of first light emitting portions 441a, 441b, and 441c has a first beam angle, the plurality of first light emitting portions 441a, 441b, and 441c may be spaced apart from each other so that light emitted by the plurality of first light emitting portions 441a, 441b, and 441c does not interfere with each other. The second light emitting portion 451 may be spaced apart from each of the plurality of first light emitting portion s 441a, 441b, and 441c to decrease crosstalk between light emitted by the second light emitting portion 451 and the plurality of first light emitting portions 441a, 441b, and 441c. When a plurality of first light emitting portions 441a, 441b, and 441c are not disposed to surround the second light emitting portion 451, a space for disposing the plurality of first light emitting portions 441a, 441b, and 441c in the electronic device 400 that is miniaturized to be worn on a part of the user's body may be insufficient. When the plurality of first light emitting portions 441a, 441b, and 441c are disposed on the printed circuit board 420 to surround the second light emitting portion 451, the electronic device 400 may arrange the required number of the plurality of first light emitting portions 441a, 441b, and 441c within the limited internal space of the electronic device 400. When the plurality of first light emitting portions 441a, 441b, and 441c are disposed to surround the second light emitting portion 451, since the distance between the at least one second light emitting portion 451 and the at least one second receiving portion 452 forming the laser sensor 450 is closer than the distance between the plurality of first light emitting portions 441a, 441b, and 441c and the first receiving portion 442 forming the PPG sensor 440, the laser sensor 450 may be disposed to be surrounded by the PPG sensor 440. According to an embodiment, since the PPG sensor 440 is disposed on the printed circuit board 420 to surround the laser sensor 450, the electronic device 400 may provide various biometric information to the user by disposing the PPG sensor 440 and the laser sensor 450 having different optical characteristics within a limited internal space of the electronic device.

According to an embodiment, the laser sensor 450 may be disposed to overlap the first region 432 of the window 430 when the window 430 is viewed from the outside of the electronic device 400. When the laser sensor 450 is not disposed in the region of the printed circuit board 420 corresponding to the first region 432 of the window 430, light emitted from the second light emitting portion 451 may not be transmitted to the user's body or the second receiving portion 452 may not receive light. According to an embodiment, the electronic device 400 may transmit light emitted from the second light emitting portion 451 to the user or may maintain light reception at the second receiving portion 452 , when the laser sensor 450 is disposed to overlap the first region 432 of the window 430 when the window 430 viewed from the outside of the electronic device 400 (i.e. in a top view). For example, when the laser sensor 450 is disposed to overlap the first region 432, the light emitted from the second light emitting portion 451 may be incident substantially perpendicularly on the first region 432 and the part of the user's body. When light emitted from the second light emitting portion 451 is substantially perpendicular to the part of the user's body to be transferred to the part of the user's body, the possibility of loss due to refraction may be reduced. In another example, when laser sensor 450 is disposed to overlap the first region 432, when the light reflected from the part of the user's body is received by the second receiving portion 452, the light may be incident on the first region 432. When light reflected from the part of the user's body substantially perpendicular to the first region 432 is received by the second receiving portion 452, the possibility of loss due to refraction may be reduced.

According to an embodiment, the PPG sensor 440 may be disposed on the printed circuit board 420 to overlap the second region 433 of the window 430 when the window 430 is viewed from the outside of the electronic device 400.

According to an embodiment, the index layer 460 may reduce a difference in refractive index between the inner space 411 and the window 430. The index layer 460 may form a path through which light is transmitted from the second light emitting portion 451 to the window 430 or a path through which light is transmitted from the window 430 to the second receiving portion 452. The index layer 460 may protect the laser sensor 450 from forces applied from the outside of the electronic device 400. For example, the index layer 460 may fill between the second light emitting portion 451 and the window 430 to reduce the difference in refractive index between the window 430 and the second light emitting portion 451 and protect the second light emitting portion 451 from external impacts. The index layer 460 may be made of a transparent material and may have elasticity. According to an embodiment, the index layer 460 may be disposed on the laser sensor 450 to overlap the laser sensor 450 when the window 430 is viewed from the outside of the electronic device 400. For example, when the index layer 460 is not used, light emitted from the second light emitting portion 451 may be dispersed, or the light received by the second receiving portion 452 may not receive light, due to the difference in refractive index between the inner space 411 and the window 430. When the light emitted from the second light emitting portion 451 is dispersed or the light is not received by the second receiving portion 452, performance of the laser sensor 450 may be degraded. According to an embodiment, the electronic device 400 may include the index layer 460 that compensates for the difference in refractive index between the internal space 411 and the window 430 to improve performance of the laser sensor 450. According to an embodiment, the index layer 460 may be made of a material that transmits light. For example, the index layer 460 may be made of at least one of polyethylene and rubber, but is not limited thereto.

As described above, since the PPG sensor 440 is disposed on the printed circuit board 420 to surround the laser sensor 450, the electronic device 400 according to an embodiment may arrange the PPG sensor 440 and the laser sensor 450 having different optical characteristics within a limited space. According to an embodiment, the electronic device 400 may provide various biometric information to the user by including the PPG sensor 440 and the laser sensor 450.

According to an embodiment, the electronic device 400 transmit light emitted from the second light emitting portion 451 to the user or maintain light reception by the second receiving portion 452 when the laser sensor 450 is disposed to overlap the first region 432 of the window 430 when the window 430 viewed from the outside of the electronic device 400.

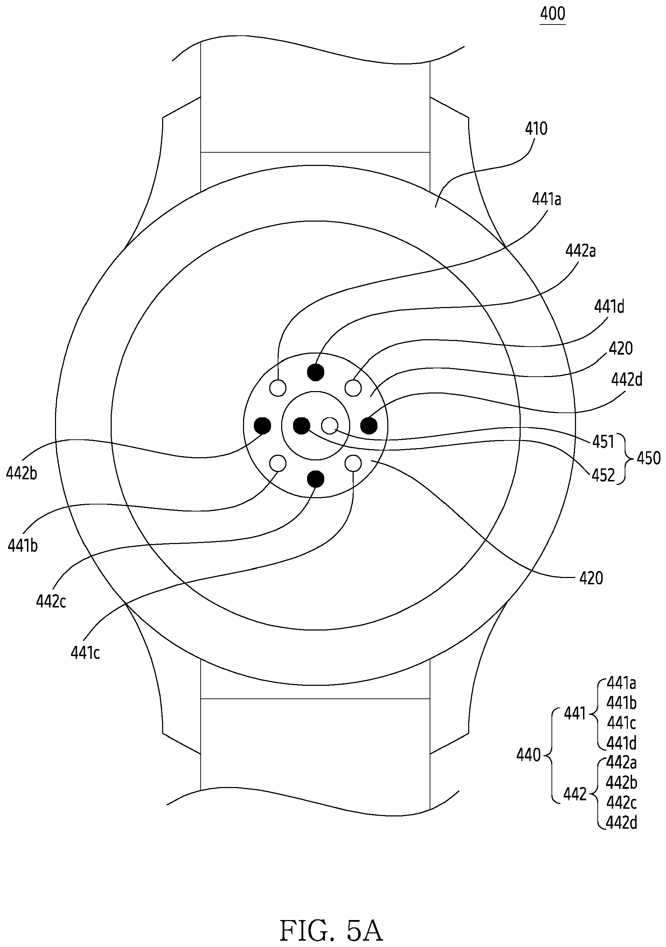

FIG. 5A is a top view illustrating an example of an arrangement relationship between a PPG sensor and a laser sensor of an electronic device according to an embodiment.

Referring to FIG. 5A, the PPG sensor 440 of the electronic device 400 according to an embodiment may include a plurality of first light emitting portions 441a, 441b, 441c, and 441d and a plurality of first receiving portions 442a, 442b, 442c, and 442d.

According to an embodiment, the outer shape of the laser sensor 450 may be substantially circular, but is not limited thereto. For example, the printed circuit board 420 may be substantially circular, and the laser sensor 450 may be disposed on the printed circuit board 420 to be adjacent to the center of the printed circuit board 420.

According to an embodiment, the PPG sensor 440 may be spaced apart from the laser sensor 450 in the radial direction of the laser sensor 450. By being spaced apart in the radial direction of the laser sensor 450, the PPG sensor 440 may surround the laser sensor 450 from the outside the laser sensor 450.

According to an embodiment, the plurality of first light emitting portions 441a, 441b, 441c, and 441d of the PPG sensor 440 may be spaced apart from each other. For example, a plurality of first light emitting portions 441a, 441b, 441c, and 441d may be spaced apart from each other along the periphery of the laser sensor 450.

According to an embodiment, the plurality of first receiving portions 442a, 442b, 442c, and 442d of the PPG sensor 440 may be spaced apart from each other. For example, each of the plurality of first receiving portions 442a, 442b, 442c, and 442d may be located in a space between the plurality of first light emitting portions 441a, 441b, 441c, and 441d along the periphery of the laser sensor 450.

As described above, since the PPG sensor 440 is disposed on the printed circuit board 420 to surround the laser sensor 450, the electronic device 400 according to an embodiment may arrange the PPG sensor 440 and the laser sensor 450 having different optical characteristics within limited space. According to an embodiment, the electronic device 400 may provide various biometric information to the user by including the PPG sensor 440 and the laser sensor 450.

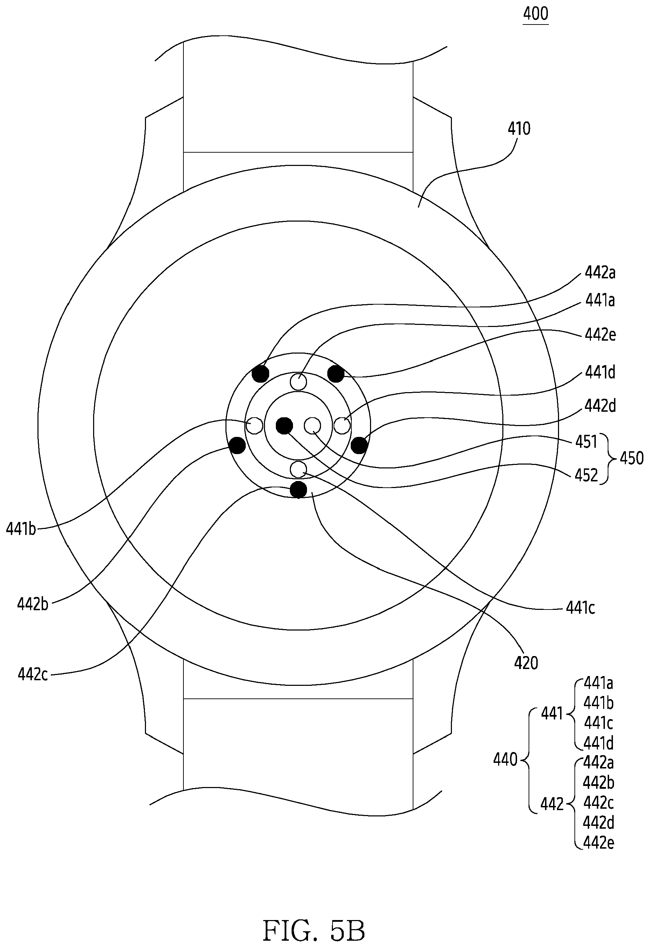

FIG. 5B is a top view illustrating another example of an arrangement relationship between a PPG sensor and a laser sensor of an electronic device, according to an embodiment.

Referring to FIG. 5B, according to an embodiment, the PPG sensor 440 of an electronic device 400 may include the plurality of first light emitting portions 441a, 441b, 441c, and 441d and the plurality of first receiving portions 442a, 442b, 442c, 442d, and 442e.

According to an embodiment, the outer shape of the laser sensor 450 may be substantially circular, but is not limited thereto. For example, the printed circuit board 420 may be substantially circular, and the laser sensor 450 may be disposed on the printed circuit board 420 to be adjacent to the center of the printed circuit board 420.

According to an embodiment, the PPG sensor 440 may be spaced apart from the laser sensor 450 in the radial direction of the laser sensor 450. By being spaced apart in the radial direction of the laser sensor 450, the PPG sensor 440 may surround the laser sensor 450 from the outside the laser sensor 450.

According to an embodiment, the plurality of first light emitting portions 441a, 441b, 441c, and 441d of the PPG sensor 440 may surround the laser sensor 450 from the outside the laser sensor 450. For example, the plurality of first light emitting portions 441a, 441b, 441c, and 441d may be spaced apart from each other along the periphery of the laser sensor 450.

According to an embodiment, the plurality of first receiving portions 442a, 442b, 442c, 442d, and 442e of the PPG sensor 440 may surround the plurality of first light emitting portions 441a, 441b, 441c, and 441d from the outside the plurality of first light emitting portions 441a, 441b, 441c, and 441d. For example, the plurality of first receiving portions 442a, 442b, 442c, 442d, and 442e may be spaced apart from each other along peripheries of the plurality of first light emitting portions 441a, 441b, 441c, and 441d.

As described above, since the PPG sensor 440 is disposed on the printed circuit board 420 to surround the laser sensor 450, the electronic device 400 according to an embodiment may arrange the PPG sensor 440 and the laser sensor 450 having different optical characteristics within limited space. According to an embodiment, the electronic device 400 may provide various biometric information to the user by including the PPG sensor 440 and the laser sensor 450.

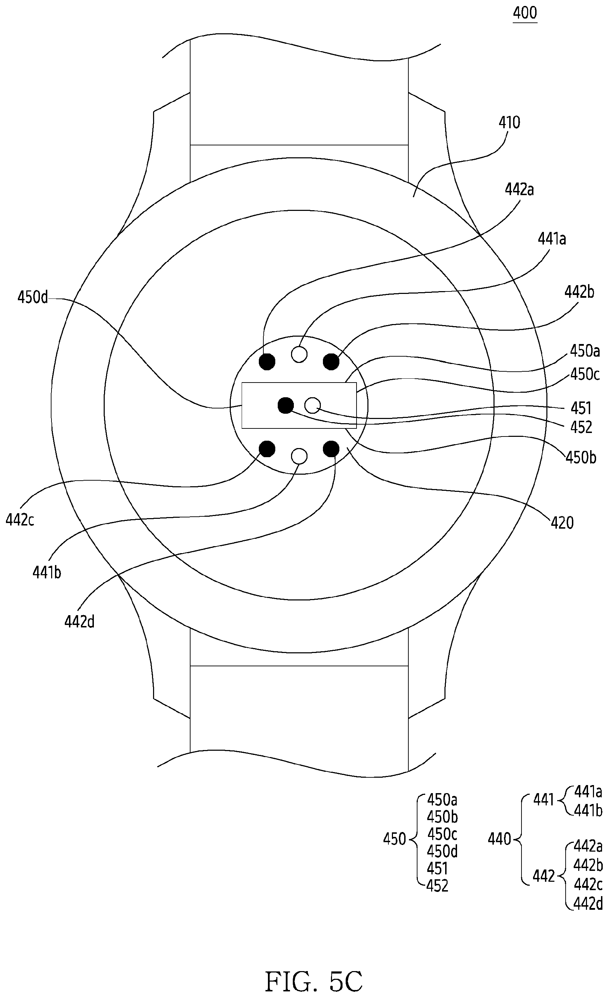

FIG. 5C is a top view illustrating yet another example of an arrangement relationship between a PPG sensor and a laser sensor of an electronic device according to an embodiment.

Referring to FIG. 5C, the PPG sensor 440 of the electronic device 400 according to an embodiment may include the plurality of first light emitting portions 441a and 441b and the plurality of first receiving portions 442a, 442b, 442c, and 442d.

According to an embodiment, the laser sensor 450 may include a plurality of long sides 450a and 450b parallel to each other and a plurality of short sides 450c and 450d that are parallel to each other and have length shorter than that of the plurality of long sides.

According to an embodiment, the plurality of first light emitting portions 441a and 441b may be disposed on the printed circuit board 420 to face each other with respect to the laser sensor 450. For example, a part 441a of the plurality of first light emitting portions 441a and 441b may face the first long side 450a, and the other part 441b of the plurality of first light emitting portions 441a and 441b may face the second long side 450b.

According to an embodiment, the plurality of first receiving portions 442a, 442b, 442c, and 442d may be disposed on the printed circuit board 420 to face each other with respect to the laser sensor 450. For example, part 442a and 442b of the plurality of first receiving portions 442a, 442b, 442c, and 442d may face the first long side 450a, and the other part 442c and 442d of the plurality of first receiving portions 442a, 442b, 442c, and 442d may face the second long side 450b.

As described above, since the PPG sensor 440 is disposed on the printed circuit board 420 to surround the laser sensor 450, the electronic device 400 according to an embodiment may arrange the PPG sensor 440 and the laser sensor 450 having different optical characteristics within limited space. According to an embodiment, the electronic device 400 may provide various biometric information to the user by including the PPG sensor 440 and the laser sensor 450.

FIG. 6 is a cross-sectional view illustrating an example in which an electronic device is cut along A-A' of FIG. 4 according to an embodiment.

Referring to FIG. 6, the window 430 of the electronic device 400 according to an embodiment may include an absorption layer 434. The absorption layer 434 may be inserted into the window 430 to absorb light passing through the window 430. For example, the absorption layer 434 may be applied to one surface 430a of the window 430 facing the printed circuit board 420. In another example, the absorption layer 434 may be interposed between one surface 430a of the window 430 facing the printed circuit board 420 and the other surface 430b of the window 430 facing the outside of the electronic device 400. Regions of the window 430 in which the absorption layer 434 is absent may be denoted as the plurality of openings 431.

According to an embodiment, when the window 430 is viewed from the outside of the electronic device 400, a plurality of openings 431 may be formed in regions of the window 430 overlapping the first light emitting portion 441, the first receiving portion 442, the second light emitting portion 451, and the second receiving portion 452.

According to an embodiment, the index layer 460 may be interposed between the window 430 and the laser sensor 450. For example, the index layer 460 may extend from one surface of the laser sensor 450 facing the window 430 to one surface of the window 430 facing the laser sensor 450.

According to an embodiment, when the electronic device 400 is worn by a user, a part B of the user's body may be deformed by the window 430 and may be in contact with the first region 432 and the second region 433. When the second region 433 has curvature, and when the electronic device 400 is worn by the user, the part B of the user's body may be deformed by the window 430 to be in close contact with the second region 433. When the part B of the user's body is in close contact with the second region 433, the electronic device 400 may be secured to the user's body so that it does not move relative to the user's body and may maintain the state of contact to the part B of the user's body. For example, when the second region 433 does not have curvature, since the electronic device 400 may not be in close contact with the part B of the user's body, the wearing position may be not be secured and may move relative to the user's body. When the electronic device 400 is not fixed, the light received by the second receiving portion 452 from the second light emitting portion 451 may include excessive noise, and thus performance of the laser sensor 450 may be degraded. According to an embodiment, the electronic device 400 may be fixed to a particular position on the user's body to ensure performance of the laser sensor 450 by having curvature to maximize the area of contact to the part B of the user's body.

According to an embodiment, the first light emitting portion 441 may emit light to the first beam angle *θ*₁ toward the part B of the user's body. The light emitted from the first light emitting portion 441 may sequentially pass through the inner space 411 and the window 430 and pass to the part B of the user's body. A part of the light transmitted to the part B of the user's body may be absorbed in the inside (e.g., blood vessels, bones, and cell tissues) of the part B of the user's body. Another part of light transmitted to the part B of the user's body may not be absorbed in the part B of the user's body, but may be reflected from the part B of the user's body. At least a part of the light reflected from either the inside or the exterior of the part B of the user's body may sequentially pass through the window 430 and the inner space 411 to be received by the first receiving portion 442. The first receiving portion 442 may obtain the first biometric data by generating an electrical signal based on the intensity or other characteristics of the received light. The electrical signal generated by the first receiving portion 442 may be received by the processor (e.g., the processor 120 of FIG. 1) through the printed circuit board 420.

According to an embodiment, the second light emitting portion 451 may emit light to a second beam angle *θ*₂ smaller than the first beam angle *θ*₁ toward the part B of the user's body. The light emitted from the second light emitting portion 451 may sequentially pass through the index layer 460 and the window 430 and may be transmitted to the part B of the user's body. A part of the light transmitted to the part B of the user's body may reach a designated material (e.g., glucose molecule or alcohol molecule) in the part B of the user's body. The wavelength of light reaching the designated material may be changed by the natural vibration (e.g., Raman scattering) of the designated material, or the intensity of light reaching the designated material may be changed by being absorbed by the designated material. The light with the changed wavelength or changed intensity may be received from the part B of the user's body through the window 430 and the index layer 460 by the second receiving portion 452. The second receiving portion 452 may obtain the second biometric data by generating an electrical signal based on the intensity of the received light or the wavelength of the received light. The electrical signal generated by the second receiving portion 452 may be received by the processor 120 through the printed circuit board 420.

As described above, since the PPG sensor 440 is disposed on the printed circuit board 420 to surround the laser sensor 450, the electronic device 400 according to an embodiment may arrange the PPG sensor 440 and the laser sensor 450 having different optical characteristics within limited space. The electronic device 400 may provide various biometric information to a user by obtaining different first biometric data and second biometric data from the PPG sensor 440 and the laser sensor 450, respectively.

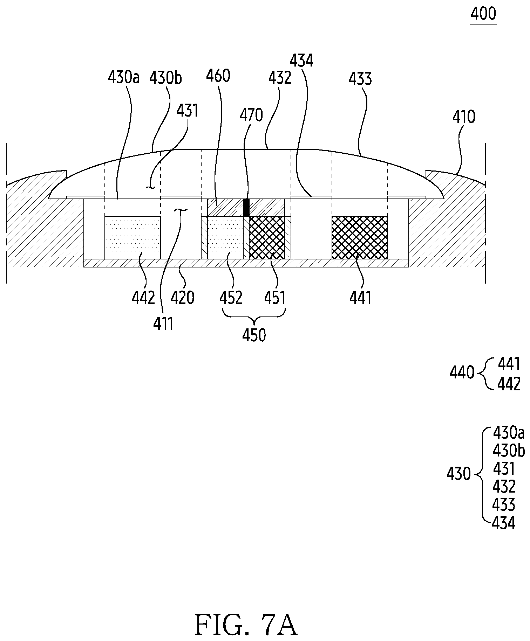

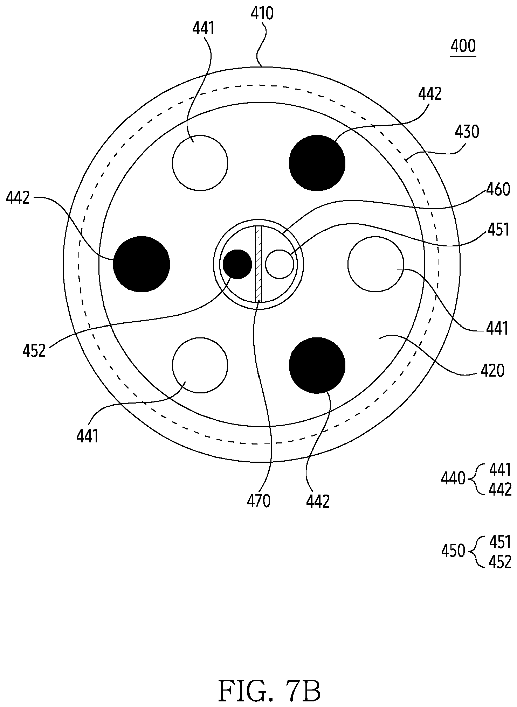

FIG. 7A is a cross-sectional view illustrating a cross-section of an electronic device according to an embodiment. FIG. 7B is a plan view of a second surface of an electronic device, according to an embodiment.

Since the electronic device 400 of FIGS. 7A and/or 7B may be similar to the electronic device 400 previously described in connection with FIG. 6, except that a barrier 470 is added, duplicative description thereof will be omitted.

Referring to FIGS. 7A and 7B, the electronic device 400 may further include a barrier 470. The barrier 470 may prevent crosstalk between light emitted from the second light emitting portion 451 and light received by the second receiving portion 452. The barrier 470 may prevent noise from occurring in the laser sensor 450 by preventing crosstalk of light between the second light emitting portion 451 and the second receiving portion 452. According to an embodiment, the barrier 470 may protect the laser sensor 450 from forces applied from the outside of the electronic device 400. For example, the barrier 470 may be made of a substantially opaque material so as not to transmit light, but is not limited thereto.

According to an embodiment, when viewed from the outside of the electronic device 400, the barrier 470 may be disposed on the laser sensor 450 to be positioned between a region overlapping the second light emitting portion 451 and a region overlapping the second receiving portion 452. For example, the barrier 470 may be located to be separate from the second light emitting portion 451 and the second receiving portion 452.

According to an embodiment, the barrier 470 may be interposed between the window 430 and the laser sensor 450. For example, the barrier 470 may be inserted into the index layer 460 so that it extends between one surface 430a of the window 430 and one surface of the laser sensor 450 facing each other.

According to the above-described embodiment, the electronic device 400 may improve the performance (e.g., signal to noise ratio (SNR)) of the laser sensor 450 by including the barrier 470 that prevents light crosstalk between the second light emitting portion 451 and the second receiving portion 452. According to an embodiment, the electronic device 400 may prevent the laser sensor 450 from being damaged by forces applied from the outside of the electronic device 400 by interposing the barrier 470 between the window 430 and the laser sensor 450.

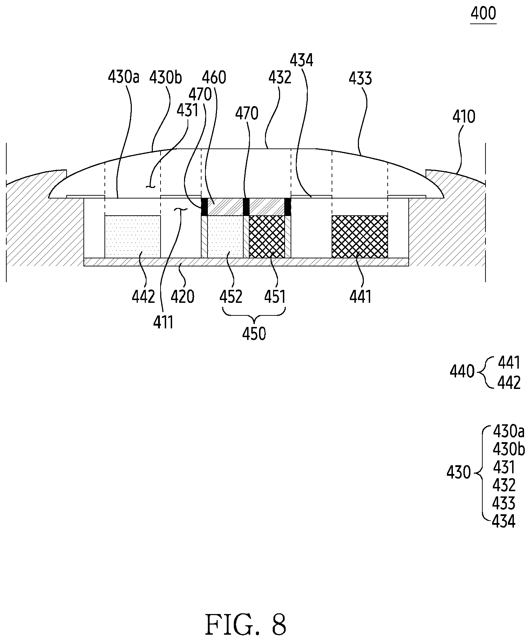

FIG. 8 is a cross-sectional view illustrating a cross-section of an electronic device according to an embodiment.

An electronic device 400 of FIG. 8 may be similar to the electronic device 400 previously described in connection with FIGS. 7A and 7B, except that the arrangement structure of the barrier 470 is changed, and thus duplicative description thereof will be omitted.

Referring to FIG. 8, the barrier 470 of the electronic device 400 according to an embodiment may surround an outer surface of the index layer 460. Since the barrier 470 surrounds the outer surface of the index layer 460, light emitted from the second light emitting portion 451 or light incident upon the second receiving portion 452 may be prevented from being emitted to the outer surface of the index layer 460. As light is blocked from transmitting to the outer surface of the index layer 460 is blocked, the performance of the laser sensor 450 may be improved.

According to the above-described embodiment, the electronic device 400 may improve the performance of the laser sensor 450 by including the barrier 470 for preventing light crosstalk between the second light emitting portion 451 and the second receiving portion 452. In the electronic device 400 according to an embodiment, as the barrier 470 surrounds the outer surface of the index layer 460, light is blocked from transmitting to the outer surface of the index layer 460, so that the performance of the laser sensor 450 may be improved.

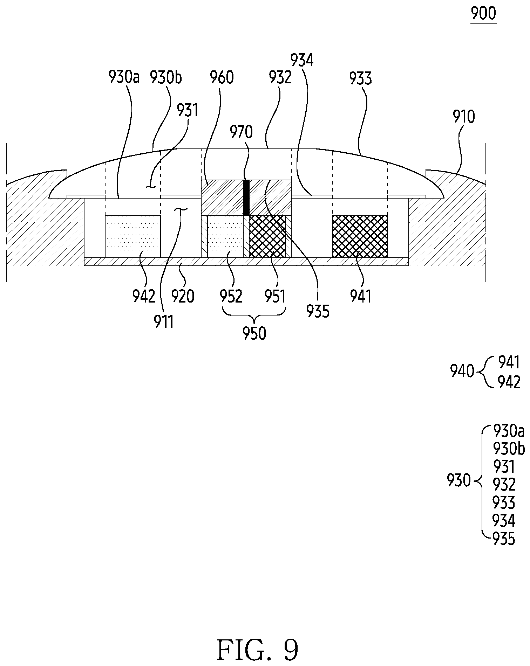

FIG. 9 is a cross-sectional view illustrating a cross-section of an electronic device according to an embodiment.

Referring to FIG. 9, the electronic device 900 may include a housing 910, a printed circuit board 920, a window 930, a PPG sensor 940, a laser sensor 950, an index layer 960, and a barrier 970. The housing 910, the printed circuit board 920, the window 930, the PPG sensor 940, the laser sensor 950, the index layer 960, and the barrier 970 of FIG. 9 may be substantially the same as the housing 410, the printed circuit board 420, the window 430, the PPG sensor 440, the laser sensor 450, the index layer 460, and the barrier 470 of FIG. 4, and duplicative descriptions thereof will be omitted.

According to an embodiment, the window 930 may include an accommodating groove 935. The accommodating groove 935 may receive the index layer 960 and the barrier 970 so as to fix their positions within the electronic device 900. According to an embodiment, the accommodating groove 935 may be formed by recessing one region of the window 930 facing the laser sensor 950 toward the outside of the electronic device 900. According to an embodiment, when the window 930 is viewed from the outside of the electronic device 900, the accommodating groove 935 may overlap the laser sensor 950.

According to an embodiment, the index layer 960 may be inserted into the accommodating groove 935. For example, the index layer 960 may extend from one surface of the laser sensor 950 facing the accommodating groove 935 into the accommodating groove 935. As the index layer 960 is seated in the accommodating groove 935, the accommodating groove 935 may prevent the index layer 960 from being separated from the designed position.

According to an embodiment, the barrier 970 may be inserted into the accommodating groove 935 and the index layer 960. For example, the barrier 970 may extend into the accommodating groove 935 from one surface of the laser sensor 950 facing the accommodating groove 935. As he barrier 970 is seated in the accommodating groove 935, the accommodating groove 935 may prevent the barrier 970 from being separated from the designed position.

According to the above-described embodiment, as the index layer 960 and the barrier 970 are seated in the accommodating groove 935 formed in the window, the electronic device 900 may prevent the index layer 960 and the barrier 970 from being separated from their designed positions.

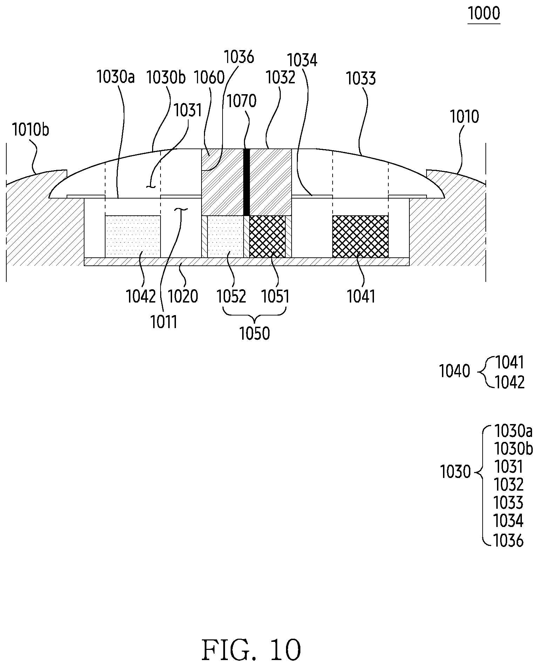

FIG. 10 is a cross-sectional view illustrating a cross-section of an electronic device according to an embodiment.

Referring to FIG. 10, the electronic device 1000 may include a housing 1010, a printed circuit board 1020, a window 1030, a PPG sensor 1040, a laser sensor 1050, an index layer 1060, and a barrier 1070. The housing 1010, the printed circuit board 1020, the window 1030, the PPG sensor 1040, the laser sensor 1050, the index layer 1060, and the barrier 1070 of FIG. 10 may be substantially the same as the housing 410, the printed circuit board 420, the window 430, the PPG sensor 440, the laser sensor 450, the index layer 460, and the barrier 470 of FIG. 4, and duplicative descriptions thereof will be omitted.

According to an embodiment, the window 1030 may include a through hole 1036. The through hole 1036 may connect the inner space 1011 of the housing 1010 and the outside of the electronic device 1000. For example, the through hole 1036 may extend from one surface 1030a of the window 1030 facing the laser sensor 1050 to the outside of the electronic device 1000.

According to an embodiment, the index layer 1060 may be inserted into the through hole 1036. For example, one surface of the index layer 1060 may be in contact with one surface of the laser sensor 450 facing the window 1030, and another surface of the index layer 1060 may be exposed to the outside of the electronic device 1000. The other surface of the index layer 1060 exposed to the outside of the electronic device 1000 may form a part of the second surface 1010b of the housing 1010. When the electronic device 1000 is worn by the user, the other surface of the index layer 1060 exposed to the outside of the electronic device 1000 may contact a part of the user's body. As the index layer 1060 is exposed from the laser sensor 1050 to the outside of the electronic device 1000, there may be no difference in refractive index in the path of light between the laser sensor 1050 and the second surface 1010b. As the difference in refractive index is eliminated, light emitted from the second light emitting portion 1051 may be transmitted to the outside of the electronic device 1000 without loss, or light reflected from the user's body may be transmitted to the second receiving portion1052 without loss.

According to an embodiment, the barrier 1070 may be inserted into the through hole 1036 and the index layer 1060. For example, one surface of the barrier 1070 may be in contact with one surface of the laser sensor 1050 facing the window 1030, and another surface of the barrier 1070 may be visually exposed to the outside of the electronic device 1000.

According to the above-described embodiment, as the index layer 1060 connects the laser sensor 1050 to the outside of the electronic device 1000, the electronic device 1000 may not have refractive index differences in the path of light between the laser sensor 1050 and the second surface 1010b. As the difference in refractive index is removed, light is transmitted from the second light emitting unit 1051 to the user's body without excessive loss, interference or noise or light is received from the user's body to the second receiving portion 1052 without excessive loss, interference or noise, thereby ensuring performance of the laser sensor 1050.

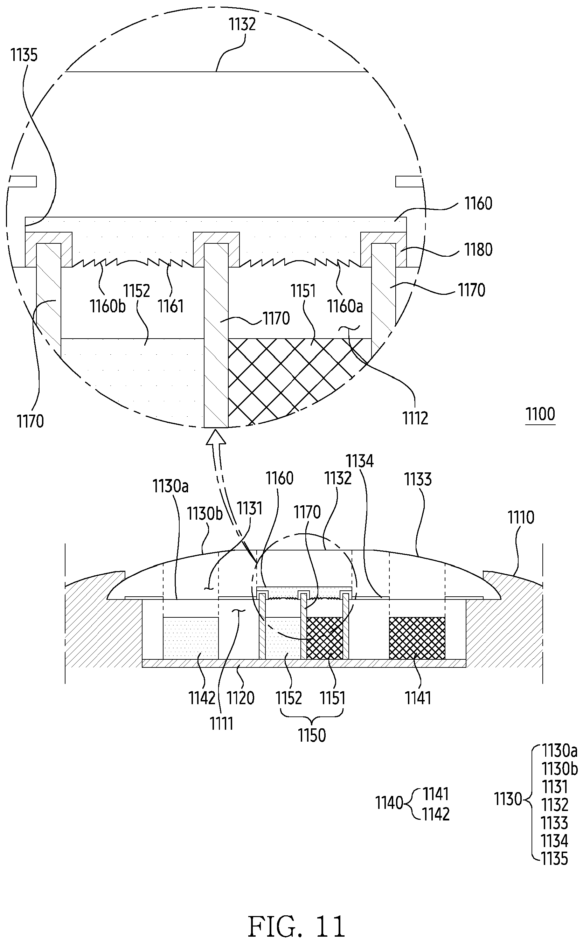

FIG. 11 is a cross-sectional view illustrating a cross-section of an electronic device according to an embodiment.

The electronic device 1100 may include a housing 1110, a printed circuit board 1120, a window 1130, a PPG sensor 1140, a laser sensor 1150, an index layer 1160, a barrier 1170 and a buffering member 1180. The housing 1110, the printed circuit board 1120, the window 1130, the PPG sensor 1140, the laser sensor 1150, the index layer 1160, and the barrier 1170 of FIG. 11 may be substantially the same as the housing 410, the printed circuit board 420, the window 430, the PPG sensor 440, the laser sensor 450, the index layer 460, and the barrier 470 of FIG. 4, and duplicative descriptions thereof will be omitted.

According to an embodiment, the window 1130 may include an accommodating groove 1135. According to an embodiment, the accommodating groove 1135 may be formed by recessing one part of the window 1130 facing the laser sensor 1150. According to an embodiment, when the window 1130 is viewed from the outside of the electronic device 1100, the accommodating groove 1135 may overlap the laser sensor 1150.

According to an embodiment, the index layer 1160 may be inserted into the accommodating groove 1135. As the index layer 1160 is seated in the accommodating groove 1135, the accommodating groove 1135 may prevent the index layer 1160 from being separated from the designed position. According to an embodiment, the index layer 1160 may be spaced apart from the laser sensor 1150. For example, the index layer 1160 may be spaced apart from the laser sensor 1150 in the direction from the printed circuit board 1120 toward the window 1130. As the index layer 1160 and the laser sensor 1150 are spaced apart from each other, an air gap 1112 may be formed between the index layer 1160 and the laser sensor 1150.

According to an embodiment, the barrier 1170 may extend from the printed circuit board 1120 to the window 1130. For example, the barrier 1170 may be inserted into the index layer 1160 in which one end is in contact with the printed circuit board 1120 and the other end is disposed within the accommodating groove 1135. According to an embodiment, the barrier 1170 may extend between the second light emitting portion 1151 and the second receiving portion 1152 to prevent crosstalk between light emitted from the second light emitting portion 1151 and light received by the second receiving portion 1152. According to an embodiment, the barrier 1170 may surround outer surfaces of the second light emitting portion 1151 and the second receiving portion 1152, and may extend to the accommodating groove 1135. As the barrier 1170 surrounds the outer surfaces of the second light emitting portion 1151 and the second receiving portion 1152, light emitted from the second light emitting portion 1151 or light proceeding to the second receiving portion 1152 may be prevented from being transferred to the inner space 1111. As the transmission of light to the inner space 1111 is prevented, the performance of the laser sensor 1150 may be improved.

In FIG. 11, the barrier 1170 is illustrated to be disposed adjacent to the laser sensor 1150 to ensure performance of the laser sensor 1150, but this is only one example. In an embodiment, the barrier 1170 disposed adjacent to the PPG sensor 1140 may be added. For example, the barrier 1170 may extend from the printed circuit board 1120 to the window 1130 to surround outer surfaces of the first light emitting portion 1141 and the first receiving portion 1142. Since the barrier 1170 is disposed adjacent to the PPG sensor 1140, performance of the PPG sensor 1140 may be improved.

According to an embodiment, the buffering member 1180 may reduce friction between the window 1130 and the barrier 1170. The buffering member 1180 may be interposed between the window 1130 and the barrier 1170. For example, the buffering member 1180 may surround one end of the barrier 1170 positioned inside the accommodating groove 1135. For example, the buffering member 1180 may be made of silicon foam, but is not limited thereto, and may be made of an elastic material.

According to an embodiment, the index layer 1160 may include a Fresnel pattern 1161. The Fresnel pattern 1161 may concentrate light. For example, the Fresnel pattern 1161 may have a collective shape of a plurality of concentric circles sharing a center. According to an embodiment, the Fresnel pattern 1161 may be formed on one surface of the index layer 1160 facing the laser sensor 1150. For example, the Fresnel pattern 1161 may be formed on one surface 1160a of the index layer 1160 facing the second light emitting portion 1151 and the other surface 1160b of the index layer 1160 facing the second receiving portion 1152. The surfaces 1160a and 1160b of the index layer 1160 on which the Fresnel pattern 1161 is formed may be referred to as Fresnel lenses. Light emitted from the second light emitting unit 1151 may be concentrated by the Fresnel pattern 1161 of one surface 1160a of the index layer 1160 and transmitted to the window 1130. Light arriving at the index layer 1160 from the window 1130 may be concentrated by the Fresnel pattern 1161 of the other surface 1160b of the index layer 1160 and received by the second receiving portion 1152.

According to an embodiment described above, since the index layer 1160 includes the Fresnel pattern 1161, the electronic device 1100 may ensure performance of the laser sensor 1150 by concentrating, by the Fresnel pattern 1161, the light emitted from the second light emitting portion 1151 or the light transmitted from the outside of the electronic device 1100 to the second receiving portion 1152.

According to an embodiment, an electronic device (e.g., the electronic device 400 of FIG. 4) may comprise a housing (e.g., housing 410 in FIG. 4), a printed circuit board (e.g., printed circuit board 420 of FIG. 4) disposed within the housing, a window (e.g., window 430 in FIG. 4) including a first region (e.g., the first region 432 of FIG. 4) parallel to the printed circuit board and a second region (e.g., the second region 433 of FIG. 4) connecting a periphery of the first region and the housing and facing a part of user's body when the electronic device is worn by the user, a PPG (photoplethysmogram) sensor (e.g., the PPG sensor 440 of FIG. 4) disposed on the printed circuit board and including at least one first emitting portion (e.g., the first light emitting portion 441 of FIG. 4) configured to emit light at a first beam angle toward the window and at least one first receiving portion (e.g., the first receiving portion 442 of FIG. 4) configured to receive light emitted from the at least one first emitting portion and reflected from the part of the user's body, a laser sensor (e.g., the laser sensor 450 of FIG. 4) including at least one second emitting portion (e.g., the second light emitting portion 451 of FIG. 4) configured to emit light at a second beam angle smaller than the first beam angle toward the window, and at least one second receiving portion (e.g., the second receiving portion 452 of FIG. 4) configured to receive light emitted from the at least one second emitting portion and reflected from the part of the user's body and an index layer (e.g., index layer 460 of FIG. 4) disposed on the laser sensor; wherein the laser sensor may be disposed on the printed circuit board to be surrounded by the PPG sensor and may overlap the first region, when the window is viewed from an outside of the electronic device.

According to an embodiment, the PPG sensor may be spaced apart from the laser sensor in a radial direction of the laser sensor.

According to an embodiment, when the window is viewed from the outside of the electronic device, the PPG sensor may overlap the second region.

According to an embodiment, the index layer may be interposed between the window and the laser sensor.

According to an embodiment, the window further may include a through-hole (e.g., through hole 1036 in FIG. 10) extending from one surface (e.g., one surface 1030a of FIG. 10) of the window facing the laser sensor to the other surface of the window exposed to the outside of the electronic device, and wherein the index layer is inserted into the through-hole, and one surface of the index layer is exposed to the outside of the electronic device.

According to an embodiment, the window further may include an accommodating groove (e.g., accommodating groove 935 in FIG. 9) defined by recessing one region of the window facing the laser sensor toward the outside of the electronic device, and wherein a part of the index layer may be inserted to the accommodating groove.

According to an embodiment, the at least one first emitting portion may include a plurality of first emitting portions spaced apart from each other along a periphery of the laser sensor, and wherein the at least one first receiving portion may be disposed between the plurality of the first emitting portions along the periphery of the laser sensor.

According to an embodiment, the at least one first emitting portion may surround the laser sensor from an outside of the laser sensor, and wherein the at least one first receiving portion may surround the at least one first emitting portion from an outside of the at least one first emitting portion.

According to an embodiment, the at least one first emitting portion may include a plurality of first emitting portions each emitting light having different wavelengths, wherein the at least one second emitting portion may include a plurality of second emitting portions each emitting light having different wavelengths, and wherein a wavelength range of light emitted by the plurality of second emitting portions may be narrower than a wavelength range of light emitted by the plurality of first emitting portions.

According to an embodiment, the electronic device may further include a barrier (e.g., the barrier 470 of FIGS. 7A and 7B) disposed on the laser sensor to be positioned between one region of the window overlapping the at least one second emitting portion and another region of the window overlapping the at least one second receiving portion, when the window is viewed from the outside of the electronic device.

According to an embodiment, the barrier may be disposed to surround an outer surface of the index layer.

According to an embodiment, the barrier may be between the at least one second receiving portion and the at least one second emitting portion, and extending from the printed circuit board to the window.

According to an embodiment, the index layer may be disposed between the barrier and the window, and spaced apart from the laser sensor, and wherein a Fresnel pattern (e.g., the Fresnel pattern 1161 of FIG. 11) may be formed on one surface of the index layer facing the laser sensor.

According to an embodiment, the electronic device further includes a buffering member (e.g., the buffering member 1180 of FIG. 11) interposed between the window and the barrier.

According to an embodiment, an electronic device (e.g., the electronic device 400 of FIG. 4) may comprise a housing (e.g., the housing 410 in FIG. 4) including a first surface (e.g., the first surface 210A of FIG. 2A), a second surface (e.g., second surface 410b of FIG. 4) opposite the first surface and an inner space (e.g., inner space 411 in FIG. 4) formed between the first surface and the second surface, a printed circuit board (e.g., the printed circuit board 420 of FIG. 4) disposed in the inner space, a window (e.g., the window 430 in FIG. 4) forming at least part of the second surface of the housing, and including a first region (e.g., the first region 432 of FIG. 4) parallel to the printed circuit board and configured to contact a part of a user's body when the electronic device is worn by the user, and a second region (e.g., the second region 433 of FIG. 4) having curvature and connecting a periphery of the first region and the housing, a PPG (photoplethysmogram) (e.g., the PPG sensor 440 of FIG. 4) sensor disposed on the printed circuit board and including at least one first emitting portion (e.g., the first light emitting portion 441 of FIG. 4) configured to emit light at a first beam angle toward the window and at least one first receiving portion (e.g., the first receiving potion 442 of FIG. 4) configured to receive light emitted from the at least one first emitting portion and reflected from the part of the user's body, a laser sensor (e.g., the laser sensor 450 of FIG. 4) including at least one second emitting portion configured to emit light at a second beam angle smaller than the first beam angle toward the window, and at least one second receiving portion configured to receive light emitted from the at least one second emitting portion and reflected from the part of the user's body, an index layer (e.g., index layer 460 of FIG. 4) disposed on the laser sensor and a barrier (e.g., the barrier 470 of FIGS. 7A and 7B) disposed on the laser sensor to be positioned between one region of the window overlapping the at least one second emitting portion (e.g., the second light emitting portion 451 of FIG. 4) and another region of the window overlapping the at least one second receiving portion (e.g., the second receiving portion 452 of FIG. 4) when the window is viewed from an outside of the electronic device; wherein the laser sensor may be disposed on the printed circuit board to be surrounded by the PPG sensor and may overlap the first region when the window is viewed from the outside of the electronic device.

According to an embodiment, the PPG sensor may be spaced apart from the laser sensor in a radial direction of the laser sensor.

According to an embodiment, when the window is viewed from the outside of the electronic device, the PPG sensor may overlap the second region.

According to an embodiment, the at least one first emitting portion may include a plurality of first emitting portions spaced apart from each other along a periphery of the laser sensor, and wherein the at least one first receiving portion may be disposed between the plurality of the first emitting portions along the periphery of the laser sensor.

According to an embodiment, the at least one first emitting portion may surround the laser sensor from an outside of the laser sensor, and wherein the at least one first receiving portion may surround the at least one first emitting portion from an outside of the at least one first emitting portion.

According to an embodiment, the at least one first emitting portion may include a plurality of first emitting portions each emitting light having different wavelengths, wherein the at least one second emitting portion may include a plurality of second emitting portions each emitting light having different wavelengths, and wherein a wavelength range of light emitted by the plurality of second emitting portions may be narrower than a wavelength range of light emitted by the plurality of first emitting portions.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101, 200, 300, 400, 900, 1000, 1100) comprising:
a housing (210, 410, 910, 1010, 1110);
a printed circuit board (380, 420, 920, 1020, 1120) disposed within the housing;
a window (430, 930, 1030, 1130) including a first region parallel to the printed circuit board and a second region connecting a periphery of the first region and the housing, the window facing a part of a user's body when the electronic device is worn by the user;
a PPG, photoplethysmogram, sensor (440, 940, 1040, 1140) disposed on the printed circuit board and including at least one first emitting portion configured to emit light at a first beam angle toward the window and at least one first receiving portion configured to receive light emitted from the at least one first emitting portion and reflected from the part of the user's body;
a laser sensor (450, 950, 1050, 1150) including at least one second emitting portion configured to emit light at a second beam angle smaller than the first beam angle toward the window, and at least one second receiving portion configured to receive light emitted from the at least one second emitting portion and reflected from the part of the user's body; and
an index layer (460, 960, 1060, 1160) disposed on the laser sensor;
wherein the laser sensor is disposed on the printed circuit board to be surrounded by the PPG sensor and overlaps the first region, when the window is viewed from an outside of the electronic device.

2. The electronic device (101, 200, 300, 400, 900, 1000, 1100) of claim 1, wherein the PPG sensor (440, 940, 1040, 1140) is spaced apart from the laser sensor (450, 950, 1050, 1150) in a radial direction of the laser sensor.

3. The electronic device (101, 200, 300, 400, 900, 1000, 1100) of according to any one of claim 1 and 2, wherein, when the window (430, 930, 1030, 1130) is viewed from the outside of the electronic device, the PPG sensor (440, 940, 1040, 1140) overlaps the second region.

4. The electronic device (101, 200, 300, 400, 900, 1000, 1100) of according to any one of claim 1 to 3, wherein the index layer (460, 960, 1060, 1160) is interposed between the window (430, 930, 1030, 1130) and the laser sensor (450, 950, 1050, 1150).

5. The electronic device (101, 200, 300, 400, 900, 1000, 1100) of according to any one of claim 1 to 4, wherein the window (430, 930, 1030, 1130) further comprises a through-hole extending from one surface of the window facing the laser sensor (450, 950, 1050, 1150) to another surface of the window exposed to the outside of the electronic device, and
wherein the index layer (460, 960, 1060, 1160) is inserted into the through-hole, and one surface of the index layer is exposed to the outside of the electronic device.

6. The electronic device (101, 200, 300, 400, 900, 1000, 1100) of according to any one of claim 1 to 5, wherein the window (430, 930, 1030, 1130) further comprises an accommodating groove defined by recessing one region of the window facing the laser sensor (450, 950, 1050, 1150) toward the outside of the electronic device, and
wherein a part of the index layer (460, 960, 1060, 1160) is inserted to the accommodating groove.

7. The electronic device (101, 200, 300, 400, 900, 1000, 1100) of according to any one of claim 1 to 6, wherein the at least one first emitting portion includes a plurality of first emitting portions spaced apart from each other along a periphery of the laser sensor (450, 950, 1050, 1150), and
wherein the at least one first receiving portion is disposed between the plurality of the first emitting portions along the periphery of the laser sensor.

8. The electronic device (101, 200, 300, 400, 900, 1000, 1100) of according to any one of claim 1 to 7, wherein the at least one first emitting portion surrounds the laser sensor (450, 950, 1050, 1150) from an outside of the laser sensor, and
wherein the at least one first receiving portion surrounds the at least one first emitting portion from an outside of the at least one first emitting portion.

9. The electronic device (101, 200, 300, 400, 900, 1000, 1100) of according to any one of claim 1 to 8, wherein the at least one first emitting portion includes a plurality of first emitting portions each emitting light having different wavelengths,
wherein the at least one second emitting portion includes a plurality of second emitting portions each emitting light having different wavelengths, and
wherein a wavelength range of light emitted by the plurality of second emitting portions is narrower than a wavelength range of light emitted by the plurality of first emitting portions.

10. The electronic device (101, 200, 300, 400, 900, 1000, 1100) of according to any one of claim 1 to 9, further comprising a barrier disposed on the laser sensor (450, 950, 1050, 1150) to be positioned between one region of the window (430, 930, 1030, 1130) overlapping the at least one second emitting portion and another region of the window overlapping the at least one second receiving portion, when the window is viewed from the outside of the electronic device.

11. The electronic device (101, 200, 300, 400, 900, 1000, 1100) of according to any one of claim 1 to 10, wherein the barrier is disposed to surround an outer surface of the index layer (460, 960, 1060, 1160).

12. The electronic device (101, 200, 300, 400, 900, 1000, 1100) of according to any one of claim 1 to 11, wherein the barrier is between the at least one second receiving portion and the at least one second emitting portion, and extends from the printed circuit board (380, 420, 920, 1020, 1120) to the window (430, 930, 1030, 1130).

13. The electronic device (101, 200, 300, 400, 900, 1000, 1100) of according to any one of claim 1 to 12, wherein the index layer (460, 960, 1060, 1160) is disposed between the barrier and the window (430, 930, 1030, 1130), and spaced apart from the laser sensor (450, 950, 1050, 1150), and
wherein a Fresnel pattern is formed on one surface of the index layer facing the laser sensor.

14. The electronic device (101, 200, 300, 400, 900, 1000, 1100) of according to any one of claim 1 to 13, further comprising a buffering member interposed between the window (430, 930, 1030, 1130) and the barrier.

15. An electronic device (101, 200, 300, 400, 900, 1000, 1100) according to claim 10, wherein:
the housing (210, 410, 910, 1010, 1110) includes a first surface, a second surface opposite the first surface and an inner space disposed between the first surface and the second surface;
the printed circuit board (380, 420, 920, 1020, 1120) is disposed in the inner space;
the window (430, 930, 1030, 1130) forms at least part of the second surface of the housing, and is configured to contact a part of a user's body when the electronic device is worn by the user, and the second region has curvature.

## Patentansprüche

1. Elektronische Vorrichtung (101, 200, 300, 400, 900, 1000, 1100), umfassend:
ein Gehäuse (210, 410, 910, 1010, 1110);
eine Leiterplatte (380, 420, 920, 1020, 1120), die innerhalb des Gehäuses angeordnet ist;
ein Fenster (430, 930, 1030, 1130) beinhaltend einen ersten Bereich parallel zu der Leiterplatte und einen zweiten Bereich, der eine Peripherie des ersten Bereichs und das Gehäuse verbindet, wobei das Fenster einem Teil des Körpers eines Benutzers zugewandt ist, wenn die elektronische Vorrichtung von dem Benutzer getragen wird;
einen PPG-, Photoplethysmogramm-, Sensor (440, 940, 1040, 1140), der auf der Leiterplatte angeordnet ist und mindestens einen ersten emittierenden Abschnitt, der dazu konfiguriert ist, Licht unter einem ersten Strahlwinkel hin zu dem Fenster zu emittieren, und mindestens einen ersten Empfangsabschnitt, der dazu konfiguriert ist, Licht zu empfangen, das von dem mindestens einen ersten emittierenden Abschnitt emittiert und von dem Teil des Körpers des Benutzers reflektiert wird, beinhaltet;
einen Lasersensor (450, 950, 1050, 1150), der mindestens einen zweiten emittierenden Abschnitt, der dazu konfiguriert ist, Licht unter einem zweiten Strahlwinkel, der kleiner als der erste Strahlwinkel ist, hin zu dem Fenster zu emittieren, und mindestens einen zweiten Empfangsabschnitt, der dazu konfiguriert ist, Licht zu empfangen, das von dem mindestens einen zweiten emittierenden Abschnitt emittiert und von dem Teil des Körpers des Benutzers reflektiert wird, beinhaltet; und
eine Indexschicht (460, 960, 1060, 1160), die auf dem Lasersensor angeordnet ist;
wobei der Lasersensor auf der Leiterplatte angeordnet ist, um von dem PPG-Sensor umgeben zu sein, und den ersten Bereich überlappt, wenn das Fenster von einer Außenseite der elektronischen Vorrichtung betrachtet wird.

2. Elektronische Vorrichtung (101, 200, 300, 400, 900, 1000, 1100) nach Anspruch 1, wobei der PPG-Sensor (440, 940, 1040, 1140) von dem Lasersensor (450, 950, 1050, 1150) in einer radialen Richtung des Lasersensors beabstandet ist.

3. Elektronische Vorrichtung (101, 200, 300, 400, 900, 1000, 1100) gemäß einem von Anspruch 1 und 2, wobei, wenn das Fenster (430, 930, 1030, 1130) von der Außenseite der elektronischen Vorrichtung betrachtet wird, der PPG-Sensor (440, 940, 1040, 1140) den zweiten Bereich überlappt.

4. Elektronische Vorrichtung (101, 200, 300, 400, 900, 1000, 1100) gemäß einem von Anspruch 1 bis 3, wobei die Indexschicht (460, 960, 1060, 1160) zwischen dem Fenster (430, 930, 1030, 1130) und dem Lasersensor (450, 950, 1050, 1150) eingeschoben ist.

5. Elektronische Vorrichtung (101, 200, 300, 400, 900, 1000, 1100) gemäß einem von Anspruch 1 bis 4, wobei das Fenster (430, 930, 1030, 1130) ferner ein Durchgangsloch umfasst, das sich von einer Oberfläche des Fensters, die dem Lasersensor (450, 950, 1050, 1150) zugewandt ist, zu einer anderen Oberfläche des Fensters, die der Außenseite der elektronischen Vorrichtung ausgesetzt ist, erstreckt, und
wobei die Indexschicht (460, 960, 1060, 1160) in das Durchgangsloch eingeführt ist und eine Oberfläche der Indexschicht der Außenseite der elektronischen Vorrichtung ausgesetzt ist.

6. Elektronische Vorrichtung (101, 200, 300, 400, 900, 1000, 1100) gemäß einem von Anspruch 1 bis 5, wobei das Fenster (430, 930, 1030, 1130) ferner eine Aufnahmenut umfasst, die durch Aussparung eines Bereichs des Fensters, der dem Lasersensor (450, 950, 1050, 1150) zugewandt ist, hin zu der Außenseite der elektronischen Vorrichtung definiert ist, und
wobei ein Teil der Indexschicht (460, 960, 1060, 1160) in die Aufnahmenut eingeführt ist.

7. Elektronische Vorrichtung (101, 200, 300, 400, 900, 1000, 1100) gemäß einem von Anspruch 1 bis 6, wobei der mindestens eine erste emittierende Abschnitt eine Vielzahl von ersten emittierenden Abschnitten beinhaltet, die entlang einer Peripherie des Lasersensors (450, 950, 1050, 1150) voneinander beabstandet sind, und
wobei der mindestens eine erste Empfangsabschnitt zwischen der Vielzahl der ersten emittierenden Abschnitte entlang der Peripherie des Lasersensors angeordnet ist.

8. Elektronische Vorrichtung (101, 200, 300, 400, 900, 1000, 1100) gemäß einem von Anspruch 1 bis 7, wobei der mindestens eine erste emittierende Abschnitt den Lasersensor (450, 950, 1050, 1150) von einer Außenseite des Lasersensors umgibt, und
wobei der mindestens eine erste Empfangsabschnitt den mindestens einen ersten emittierenden Abschnitt von einer Außenseite des mindestens einen ersten emittierenden Abschnitts umgibt.

9. Elektronische Vorrichtung (101, 200, 300, 400, 900, 1000, 1100) gemäß einem von Anspruch 1 bis 8, wobei der mindestens eine erste emittierende Abschnitt eine Vielzahl von ersten emittierenden Abschnitten beinhaltet, die jeweils Licht mit verschiedenen Wellenlängen emittieren,
wobei der mindestens eine zweite emittierende Abschnitt eine Vielzahl von zweiten emittierenden Abschnitten beinhaltet, die jeweils Licht mit verschiedenen Wellenlängen emittieren, und
wobei ein Wellenlängenspektrum von Licht, das von der Vielzahl zweiter emittierender Abschnitte emittiert wird, schmaler ist als ein Wellenlängenspektrum von Licht, das von der Vielzahl erster emittierender Abschnitte emittiert wird.

10. Elektronische Vorrichtung (101, 200, 300, 400, 900, 1000, 1100) gemäß einem von Anspruch 1 bis 9, ferner umfassend eine Barriere, die auf dem Lasersensor (450, 950, 1050, 1150) angeordnet ist, um zwischen einem Bereich des Fensters (430, 930, 1030, 1130), der den mindestens einen zweiten emittierenden Abschnitt überlappt, und einem anderen Bereich des Fensters, der den mindestens einen zweiten Empfangsabschnitt überlappt, positioniert zu sein, wenn das Fenster von der Außenseite der elektronischen Vorrichtung betrachtet wird.

11. Elektronische Vorrichtung (101, 200, 300, 400, 900, 1000, 1100) gemäß einem von Anspruch 1 bis 10, wobei die Barriere angeordnet ist, um eine Außenoberfläche der Indexschicht (460, 960, 1060, 1160) zu umgeben.

12. Elektronische Vorrichtung (101, 200, 300, 400, 900, 1000, 1100) gemäß einem von Anspruch 1 bis 11, wobei die Barriere zwischen dem mindestens einen zweiten Empfangsabschnitt und dem mindestens einen zweiten emittierenden Abschnitt ist und sich von der Leiterplatte (380, 420, 920, 1020, 1120) zu dem Fenster (430, 930, 1030, 1130) erstreckt.

13. Elektronische Vorrichtung (101, 200, 300, 400, 900, 1000, 1100) gemäß einem von Anspruch 1 bis 12, wobei die Indexschicht (460, 960, 1060, 1160) zwischen der Barriere und dem Fenster (430, 930, 1030, 1130) angeordnet und von dem Lasersensor (450, 950, 1050, 1150) beabstandet ist, und
wobei ein Fresnel-Muster auf einer Oberfläche der dem Lasersensor zugewandten Indexschicht gebildet ist.

14. Elektronische Vorrichtung (101, 200, 300, 400, 900, 1000, 1100) gemäß einem von Anspruch 1 bis 13, ferner umfassend ein Pufferelement, das zwischen dem Fenster (430, 930, 1030, 1130) und der Barriere eingeschoben ist.

15. Elektronische Vorrichtung (101, 200, 300, 400, 900, 1000, 1100) gemäß Anspruch 10, wobei:
das Gehäuse (210, 410, 910, 1010, 1110) eine erste Oberfläche, eine zweite Oberfläche gegenüber der ersten Oberfläche und einen Innenraum, der zwischen der ersten Oberfläche und der zweiten Oberfläche angeordnet ist, beinhaltet;
die Leiterplatte (380, 420, 920, 1020, 1120) in dem Innenraum angeordnet ist;
das Fenster (430, 930, 1030, 1130) mindestens einen Teil der zweiten Oberfläche des Gehäuses bildet und dazu konfiguriert ist, einen Teil des Körpers eines Benutzers zu berühren, wenn die elektronische Vorrichtung vom Benutzer getragen wird, und der zweite Bereich eine Krümmung aufweist.

## Revendications

1. Dispositif électronique (101, 200, 300, 400, 900, 1000, 1100) comprenant :
un boîtier (210, 410, 910, 1010, 1110) ;
une carte de circuit imprimé (380, 420, 920, 1020, 1120) disposée à l'intérieur du boîtier ;
une fenêtre (430, 930, 1030, 1130) comprenant une première région parallèle à la carte de circuit imprimé et une seconde région reliant une périphérie de la première région et le boîtier, la fenêtre faisant face à une partie du corps d'un utilisateur lorsque le dispositif électronique est porté par l'utilisateur ;
un capteur PPG, photopléthysmogramme (440, 940, 1040, 1140) disposé sur la carte de circuit imprimé et comprenant au moins une première partie émettrice conçue pour émettre de la lumière selon un premier angle de faisceau vers la fenêtre et au moins une première partie réceptrice conçue pour recevoir de la lumière émise par l'au moins une première partie émettrice et réfléchie par la partie du corps de l'utilisateur ;
un capteur laser (450, 950, 1050, 1150) comprenant au moins une seconde partie émettrice conçue pour émettre de la lumière selon un second angle de faisceau inférieur au premier angle de faisceau vers la fenêtre, et au moins une seconde partie réceptrice conçue pour recevoir de la lumière émise par l'au moins une seconde partie émettrice et réfléchie par la partie du corps de l'utilisateur ; et
une couche d'indice (460, 960, 1060, 1160) disposée sur le capteur laser ;
dans lequel le capteur laser est disposé sur la carte de circuit imprimé pour être entouré par le capteur PPG et chevauche la première région, lorsque la fenêtre est vue depuis un extérieur du dispositif électronique.

2. Dispositif électronique (101, 200, 300, 400, 900, 1000, 1100) de la revendication 1, dans lequel le capteur PPG (440, 940, 1040, 1140) est espacé du capteur laser (450, 950, 1050, 1150) dans une direction radiale du capteur laser.

3. Dispositif électronique (101, 200, 300, 400, 900, 1000, 1100) selon l'une quelconque des revendications 1 et 2, dans lequel, lorsque la fenêtre (430, 930, 1030, 1130) est vue depuis l'extérieur du dispositif électronique, le capteur PPG (440, 940, 1040, 1140) chevauche la seconde région.

4. Dispositif électronique (101, 200, 300, 400, 900, 1000, 1100) selon l'une quelconque des revendications 1 à 3, dans lequel la couche d'indice (460, 960, 1060, 1160) est interposée entre la fenêtre (430, 930, 1030, 1130) et le capteur laser (450, 950, 1050, 1150).

5. Dispositif électronique (101, 200, 300, 400, 900, 1000, 1100) selon l'une quelconque des revendications 1 à 4, dans lequel la fenêtre (430, 930, 1030, 1130) comprend en outre un trou traversant s'étendant d'une surface de la fenêtre faisant face au capteur laser (450, 950, 1050, 1150) à une autre surface de la fenêtre exposée à l'extérieur du dispositif électronique, et
dans lequel la couche d'indice (460, 960, 1060, 1160) est insérée dans le trou traversant, et une surface de la couche d'indice est exposée à l'extérieur du dispositif électronique.

6. Dispositif électronique (101, 200, 300, 400, 900, 1000, 1100) selon l'une quelconque des revendications 1 à 5, dans lequel la fenêtre (430, 930, 1030, 1130) comprend en outre une rainure de logement définie par l'évidement d'une région de la fenêtre faisant face au capteur laser (450, 950, 1050, 1150) vers l'extérieur du dispositif électronique, et
dans lequel une partie de la couche d'indice (460, 960, 1060, 1160) est insérée dans la rainure de logement.

7. Dispositif électronique (101, 200, 300, 400, 900, 1000, 1100) selon l'une quelconque des revendications 1 à 6, dans lequel l'au moins une première partie émettrice comprend une pluralité de premières parties émettrices espacées les unes des autres le long d'une périphérie du capteur laser (450, 950, 1050, 1150), et
dans lequel l'au moins une première partie de réception est disposée entre la pluralité des premières parties émettrices le long de la périphérie du capteur laser.

8. Dispositif électronique (101, 200, 300, 400, 900, 1000, 1100) selon l'une quelconque des revendications 1 à 7, dans lequel l'au moins une première partie émettrice entoure le capteur laser (450, 950, 1050, 1150) depuis un extérieur du capteur laser, et
dans lequel l'au moins une première partie réceptrice entoure l'au moins une première partie émettrice depuis un extérieur de l'au moins une première partie émettrice.

9. Dispositif électronique (101, 200, 300, 400, 900, 1000, 1100) selon l'une quelconque des revendications 1 à 8, dans lequel l'au moins une première partie émettrice comprend une pluralité de premières parties émettrices, chacune émettant de la lumière comportant des longueurs d'onde différentes,
dans lequel l'au moins une seconde partie émettrice comprend une pluralité de secondes parties émettrices émettant chacune de la lumière comportant des longueurs d'onde différentes, et
dans lequel une plage de longueurs d'onde de lumière émise par la pluralité de secondes parties émettrices est plus étroite qu'une plage de longueurs d'onde de lumière émise par la pluralité de premières parties émettrices.

10. Dispositif électronique (101, 200, 300, 400, 900, 1000, 1100) selon l'une quelconque des revendications 1 à 9, comprenant en outre une barrière disposée sur le capteur laser (450, 950, 1050, 1150) pour être positionnée entre une région de la fenêtre (430, 930, 1030, 1130) chevauchant l'au moins une seconde partie émettrice et une autre région de la fenêtre chevauchant l'au moins une seconde partie réceptrice, lorsque la fenêtre est vue depuis l'extérieur du dispositif électronique.

11. Dispositif électronique (101, 200, 300, 400, 900, 1000, 1100) selon l'une quelconque des revendications 1 à 10, dans lequel la barrière est disposée pour entourer une surface extérieure de la couche d'indice (460, 960, 1060, 1160).

12. Dispositif électronique (101, 200, 300, 400, 900, 1000, 1100) selon l'une quelconque des revendications 1 à 11, dans lequel la barrière se situe entre l'au moins une seconde partie de réception et l'au moins une seconde partie d'émission, et s'étend depuis la carte de circuit imprimé (380, 420, 920, 1020, 1120) jusqu'à la fenêtre (430, 930, 1030, 1130).

13. Dispositif électronique (101, 200, 300, 400, 900, 1000, 1100) selon l'une quelconque des revendications 1 à 12, dans lequel la couche d'indice (460, 960, 1060, 1160) est disposée entre la barrière et la fenêtre (430, 930, 1030, 1130), et espacée du capteur laser (450, 950, 1050, 1150), et
dans lequel un motif de Fresnel est formé sur une surface de la couche d'indice faisant face au capteur laser.

14. Dispositif électronique (101, 200, 300, 400, 900, 1000, 1100) selon l'une quelconque des revendications 1 à 13, comprenant en outre un élément tampon interposé entre la fenêtre (430, 930, 1030, 1130) et la barrière.

15. Dispositif électronique (101, 200, 300, 400, 900, 1000, 1100) selon la revendication 10, dans lequel :
le boîtier (210, 410, 910, 1010, 1110) comprend
une première surface, une seconde surface opposée à la première surface et un espace interne disposé entre la première surface et la seconde surface ;
la carte de circuit imprimé (380, 420, 920, 1020, 1120) est disposée dans l'espace interne ;
la fenêtre (430, 930, 1030, 1130) forme au moins une partie de la seconde surface du boîtier, et est conçue pour entrer en contact avec une partie du corps d'un utilisateur lorsque le dispositif électronique est porté par l'utilisateur, et la seconde région comporte une courbure.
